# EUROPÄISCHE PATENTSCHRIFT

(11) **EP 1 796 563 B1**
(45) Veröffentlichungstag und Bekanntmachung des Hinweises auf die Patenterteilung: **28.08.2013**
(21) Anmeldenummer: 05714719.1
(22) Anmeldetag: 26.03.2005
(51) Int. Cl.: A61B 17/88

(54) **DREHMOMENTSCHLÜSSEL ALS RATSCHENINSTRUMENT FÜR DIE MEDIZINALTECHNIK**
TORQUE KEY AS A RATCHET INSTRUMENT IN THE MEDICAL FIELD
CLE DYNAMOMETRIQUE EN TANT QU'INSTRUMENT A CLIQUET POUR LA TECHNIQUE MEDICALE

(30) Priorität: 13.09.2004 DE 202004014195 U
(43) Veröffentlichungstag der Anmeldung: 20.06.2007
(73) Patentinhaber: THOMMEN MEDICAL AG, 4437 Waldenburg (CH)
(72) Erfinder: BAUMGARTNER, Reto, CH-4412 Nuglar (CH)
(74) Vertreter: Ullrich, Gerhard
(86) Internationale Anmeldenummer: PCT/CH2005/000177
(87) Internationale Veröffentlichungsnummer: WO 2006/029542

(56) Entgegenhaltungen:
- CH-A- 91 638
- DE-U1- 9 402 607
- FR-A- 1 498 385
- GB-A- 1 104 501
- US-A- 5 734 113
- US-A- 6 109 150

## Beschreibung

### Anwendungsgebiet der Erfindung

Die Erfindung betrifft einen Drehmomentschlüssel, welcher als Ratscheninstrument zur Anwendung in der Medizinaltechnik, insbesondere in der Dentalprothetik, ausgebildet ist. Bei in den Kieferknochen eingesetzten Implantaten werden darauf mittels spezieller Schrauben Suprastrukturen befestigt. Die Zuverlässigkeit der Verschraubungen bedingt, dass diese mit dem jeweils vorgesehenen Drehmoment ausgeführt werden. Ein zu schwaches Anziehen mit einem zu geringen Drehmoment kann zu einer selbständigen Lockerung der Verschraubung führen. Ein Überschreiten des optimalen Drehmoments, d.h. ein zu starkes Anziehen überlastet die eingesetzten Verbindungselemente und erhöht deren Bruchgefahr.

### Stand der Technik

Aus dem Maschinenbau sind Drehmomentschlüssel mit einem Biegestab und einer ziffer- oder zumindest grössenmässigen Anzeige des ausgeübten Anzugsmoments bekannt, z.B. gemäss FR 1 498 385; US 2,447,109; US 2,936,661 und US 3,587,307. Diese Werkzeuge sind jedoch nicht durch blosses Miniaturisieren als Instrumente in der Medizinaltechnik einsetzbar. In der US 5,597,305 wird ein speziell für die Dentalprothetik geeigneter Drehmomentschlüssel beschrieben, der jedoch nicht im reversiblen Ratschenmodus betrieben werden kann. Bei Spiekermann, H.: Farbatlanten der Zahnmedizin, Bd. 10 Implantologie, Georg Thieme Verlag, Stuttgart 1994, Seiten 33 und 53, werden Drehmomentschlüssel mit Ratschenfunktion offenbart, denen es jedoch an einer Anzeige oder einer Drehmoment-Begrenzung fehlt, bzw. ist die Begrenzung des Anzugsmoments mittels einer relativ kompliziert beschaffenen Rutschkupplung realisiert. Die Reibungsverhältnisse verändern sich bei diesen Instrumenten rasch in unbestimmter Weise aufgrund aufgerauhter Oberflächen. Daher sind diese Instrumente sehr empfindlich und besitzen Bauteile, welche sich nur mit übermässigem Aufwand reinigen lassen.

In der EP 0 704 281 B1 wird ein Drehmomentschlüssel mit einem Biegestab und einer ziffermässigen Anzeige des ausgeübten Anzugsmoments vorgeschlagen, der sich auch im Ratschenmodus nutzen lässt. Auch dieses Instrument besteht aus einer Mehrzahl von Einzelteilen, was vor dem Reinigen die Demontage erfordert.

Aus den US 5,653,151; US 5,709,137; US 5,996,453 sind weitere Drehmomentschlüssel bekannt, die auch den Ratschenmodus erlauben. Im Ratschenkopf ist eine etwa ovale Aufnahme vorgesehen, in der ein kleineres Sternenrad mit einem mittigen Mehrkant zum Werkzeugeinstecken liegt. An der Peripherie der Aufnahme befindet sich ein gegen das Sternenrad drückendes Federelement. Gegenüber dem Federelement hat die Aufnahme am Rand eine abschnittsweise Verzahnung, komplementär zur Verzahnung des Sternenrads. In Schraubrichtung greift die Verzahnung des Sternenrads in die Verzahnung der Aufnahme, so dass ein eingestecktes Werkzeug mitdreht. Bei der rückläufigen Ratschenbewegung gerät das Sternenrad ausser Eingriff mit der Randverzahnung und wird hierbei gegen das Federelement gedrückt. Bei der US 6,382,051 ist in der kreisrunden Aufnahme des Instrumentenkopfes ein äusserlich verzahnter Ratschenkopf eingesetzt. Peripher zur Aufnahme ist eine Kavität vorgesehen, von der sich ein Sackloch in den Ratschenkopf erstreckt. Im Sackloch steckt das erste Ende einer Schraubenfeder, deren dickeres Mittelteil vor der Sacklochmündung liegt und deren dünneres zweites Ende ein gegen die Feder bewegliches Blockierelement trägt. Das Blockierelement liegt in der Kavität, greift jedoch mit seiner äusseren Verzahnung in die Verzahnung des Ratschenkopfes ein. Bei der Schraubbewegung arretiert das Blockierelement den Ratschenkopf, während bei der rückläufigen Ratschenbewegung das Blockierelement ausser Eingriff kommt. Diese Instrumente benötigen einen äusserlich verzahnten Ratschenkopf, der sich in einer ovalen Öffnung verlagern kann oder der von einem federgelagerten Keil blockiert bzw. im Ratschenmodus freigegeben wird.

Schliesslich ist in der US 6,109,150 ein Drehmomentschlüssel mit Ratschenmechanik offenbart, bei dem das an einem Biegestab zuvorderst angeordnete Klinkensegment in die zirkuläre Verzahnung am Aussenumfang eines Ratschenrades eingreift. Im Zentrum des Ratschenrades ist eine Aufnahmeöffnung zum Einstecken des Eindrehinstruments angeordnet. Benachbart zum Biegestab verläuft ein Führungsstab, zwischen denen ein Transferast liegt. Bei Kraftausübung am Instrumentengriff verschiebt sich der Transferast axial und überträgt seine Positionsänderung auf einen Zeiger, um das generierte Drehmoment darzustellen.

### Aufgabe der Erfindung

Angesichts der als unvollkommen anzusehenden konstruktiven und funktionellen Merkmale der gegenwärtig verfügbaren Drehmomentschlüssel für die Medizinaltechnik liegt der Erfindung die Aufgabe zugrunde, ein Instrument zu schaffen, welches sich bei reduziertem Aufwand für Demontage vereinfacht reinigen lässt. Zugleich soll damit der anschliessende Aufwand für die erneute Montage vermindert werden. Die geometrischen Abmessungen und die Form des Instruments müssen die Handhabung an den verschiedenen Positionen, im engen Raum, z.B. im Mund eines Patienten problemlos ermöglichen, was die reversible Betätigung im Ratschenmodus erfordert. Der Drehmomentschlüssel muss für die Verwendung mit herkömmlichen Eindrehwerkzeugen ausgelegt sein. Die individuelle Handhabung und fortschreitende Oberflächenrauhigkeit dürfen sich nicht fehlerhaft auswirken. Eine weitere Aufgabe besteht darin, die generierten Drehmomente kontrollieren und reproduzieren zu können, so dass jedes momentan ausgeübte Anzugsmoment wertmässig ablesbar sein muss. Schliesslich soll sich der Drehmomentschlüssel zu effizienten Herstellungskosten in Serie produzieren lassen.

### Übersicht über die Erfindung

Der Drehmomentschlüssel ist als Ratscheninstrument für die Medizinaltechnik konzipiert und hat einen zuvorderst liegenden Kopfbereich, einen anschliessenden Halsbereich, dem ein Schaftbereich folgt, und einen zuhinterst angeordneten Griffbereich. Diese Bereiche erstrecken sich im Prinzip in einer gemeinsamen Ebene. Im Kopfbereich ist eine Aufnahmeöffnung vorgesehen, die von einer Umfassung umgeben ist, welche einen Mittelpunkt besitzt, durch den sich eine Achse erstreckt. Die Aufnahmeöffnung dient zum Einsetzen eines herkömmlichen Eindrehinstruments in der Erstreckung der Achse, welche zur Ebene zumindest im Prinzip senkrecht steht. An der Peripherie der Aufnahmeöffnung ist ein begrenzt bewegliches Klinkensegment angeordnet, dessen Frontpartie zur Aufnahmeöffnung weist. Die Frontpartie ist dazu bestimmt, bei Betätigung des Drehmomentschlüssels in *Vonwärtsrichtung -* also im Einschraubmodus - mit einer am Kopf des Eindrehinstruments vorhandenen Aussenkontur in mitnehmenden Eingriff zu kommen. Bei Betätigung des Drehmomentschlüssels in *Rückwärtsrichtung -* also im Ratschenmodus - löst sich der mitnehmende Eingriff zwischen der Frontpartie des Klinkensegments und der am Kopf des Eindrehinstruments vorhandenen Aussenkontur. Längs des Drehmomentschlüssels verläuft von dessen Halsbereich ein biegesteifer Basisast. Die erfinderischen Kennzeichen bestehen in den Merkmalen, wonach:
- ein auslenkbarer, vorzugsweise linear-elastischer Biegeast vorgesehen ist, über den man bei Betätigung des Drehmomentschlüssels in *Vorwärtsrichtung* das zu generierende Drehmoment mittels einer vom Benutzer ausgeübten Vorwärtskraft einbringt;
- sich vom Klinkensegment eine Klinkenfeder in den Halsbereich hinein erstreckt, und Klinkensegment sowie Klinkenfeder eine einteilige oder aus mehreren Teilen zusammengesetzte Klinke bilden;
- das Klinkensegment und die Klinkenfeder in einem kanalförmigen Spielraum angeordnet sind, welcher die Auslenkung beider in der Ebene gegen die Kraft der Klinkenfeder erlaubt; und
- die Klinke einstückig aus dem Halsbereich herausgeformt oder als separates Bauteil daran befestigt ist.

Die nachfolgenden Merkmale stellen vorteilhafte Ausführungsformen der Erfindung dar: Das Klinkensegment weist an seiner Frontpartie eine Frontnase auf, von der einerseits eine zweite Flanke und andererseits eine dritte Flanke liegen. Der Spielraum hat im Bereich der Einmündung einen ersten und einen zweiten Anschlag. Der erste Anschlag ist der zweiten Flanke gegenüber angeordnet. Bei Betätigung des Drehmomentschlüssels in *Vorwärtsrichtung* steht die zweite Flanke der zumindest im Prinzip nicht ausgelenkten Klinke in am ersten Anschlag an. Die Frontnase nimmt dabei zur Achse zumindest im Prinzip den Minimalabstand ein. Der zweite Anschlag ist zumindest anteilig der dritten Flanke gegenüber angeordnet. Bei Betätigung des Drehmomentschlüssels in *Rückwärtsrichtung* hat sich die dritte Flanke der ausgelenkten Klinke den zweiten Anschlag angenähert oder steht daran an. Nun steht die Frontnase im Maximalabstand zur Achse, die zweite Flanke ist vom ersten Anschlag entfernt positioniert und die Klinkenfeder ist gegen deren Rückstellkraft elastisch deformiert.

Die Klinkenfeder hat die Gestalt einer geraden oder zumindest partiell bogenförmigen Blattfeder und geht einerseits in einem Federabgang an das Klinkensegment und andererseits in einer Federmündung in den Halsbereich über. An die Frontnase des Klinkensegments setzt in einer Richtung eine erste Flanke an, wobei sich daran benachbart die zweite Flanke anschliesst. In der anderen Richtung setzt an die Frontnase die dritte Flanke an, welche sich abgerundet in Richtung des Federabgangs erstreckt. Die Frontnase ist als eine in Richtung der Aufnahmeöffnung erhabene Spitze oder Auswölbung ausgebildet. Der erste Anschlag ist als zumindest im Prinzip gerade Kante und der zweite Anschlag muldenförmig beschaffen. Die den Spielraum durchragende Klinkenfeder teilt einen ersten und einen zweiten Federfreiraum ab, deren Proportionen sich mit der Bewegung der Klinkenfeder ändern und in einem Boden enden, an den die Federmündung ansetzt.

Der Basisast erstreckt sich zunächst von einem im Halsbereich gelegenen Übergang als Längsschenkel über den Schaftbereich bis in einen Indikatorbereich hinein und endet frei im Griffbereich mit einem Basisgriff. Der Biegeast verläuft von einem im Halsbereich gelegenen Übergang im Prinzip parallel zum Längsschenkel des Basisasts zunächst ebenfalls als langgestreckter Längsschenkel bis in den Indikatorbereich hinein und endet frei im Griffbereich mit einem Griffteil, welches den Basisgriff überragt. Der Biegeast kann an seiner Oberseite, dem Basisast zugewandt, eine Vertiefung aufweisen, in welcher der Anschlagschenkel und der Basisgriff partiell eingebettet sind. Zwischen dem Längsschenkel des Basisasts und dem Längsschenkel des Biegeasts liegt ein Freischnitt, der sich von einem an die beiden Übergänge angrenzenden Kehlgrund bis in den Indikatorbereich hinein erstreckt.

Der Indikatorbereich wird gebildet von zumindest einer Abzweigung des Basisasts, die zwischen dem Längsschenkel und dem Basisgriff liegt, sowie von zumindest einer Abzweigung des Biegeasts, die zwischen dessen Längsschenkel und dem Griffteil liegt. Mit Betätigung des Drehmomentschlüssels in *Vorwärtsrichtung* und der Auslenkung des Biegeasts erfährt die Relativlage zwischen der zumindest einen Abzweigung des Basisasts und der zumindest einen Abzweigung des Biegeasts - ausgehend von der *Ruhestellung* mit einer *Nullposition -* eine inkrementale Änderung, welche ein Mass für das generierte Drehmoment ist. Auf der zumindest einen Abzweigung des Basisasts und der zumindest einen Abzweigung des Biegeasts sind eine erste bzw. eine zweite Messmarkierung angebracht, an welchen die Auslenkung des Biegeasts infolge der einwirkenden Vorwärtskraft als erzeugtes Drehmoment ablesbar ist.

Alternativ besteht der Indikatorbereich zunächst aus einem vom Längsschenkel sich zum Basisgriff erstreckenden Bügel, welcher von der zumindest einen Abzweigung gebildet wird und einen Freiraum umschliesst, zu dem ein Durchgang vorhanden ist. Ferner besteht der Indikatorbereich bei dieser Ausführungsform aus der zumindest einen Abzweigung des Biegeasts, welche durch den Durchgang in den Freiraum hinein ragt. Der Bügel setzt sich mäanderartig zusammen aus dem vom Längsschenkel L-förmig abbiegenden ersten Basisabzweig und dem sich daran nacheinander anschliessenden zweiten Basisabzweig und dritten Basisabzweig, der L-förmig in den Basisgriff übergeht. An den ersten Abzweig schliesst L-förmig ein zweiter Abzweig an. Ein Anschlagschenkel erstreckt sich vom Basisgriff nach innen - in Richtung des Längsschenkels - und begrenzt den Durchgang einerseits. Der Freiraum, der Anschlagschenkel und der Durchgang sind als quasi ortsfeste Elemente und der erste Abzweig mit dem zweiten Abzweig als mit dem Biegeast auslenkbare Elemente so dimensioniert, dass die Maximalauslenkung des Biegeasts durch das Aufsetzen des zweiten Abzweigs am Anschlagschenkel definiert ist. Die erste Messmarkierung ist in Gestalt einer Massskala - z.B. mit dem Bereich 0 Ncm bis 40 Ncm - zumindest auf einem der Basisabzweige, vorzugsweise auf dem zum Basisgriff benachbarten Basisabzweig, vorgesehen. Die zweite Messmarkierung ist in Gestalt eines Zeigerelements - z.B. als Bezugsstrich oder Pfeil - auf dem ersten oder zweiten Abzweig des Biegeasts, vorzugsweise auf dem frei endenden zweiten Abzweig, angebracht. In Umkehrung dazu kann die erste Messmarkierung in Gestalt eines Zeigerelements, z.B. als Bezugsstrich oder Pfeil, auf dem Anschlagschenkel des Basisastes, in Richtung des ersten Abzweigers des Biegeastes angebracht sein, wobei dann die zweite Messmarkierung in Gestalt einer Massskala, z.B. mit dem Bereich 0 Ncm bis 40 Ncm, auf dem 1. Abzweig vorgesehen ist.

Der gesamte Drehmomentschlüssel mit Kopf- und Halsbereich, Klinke, Schaft-, Indikator- und Griffbereich, Basis- und Biegeast aus einem Stück hergestellt ist. Alternativ kann die Klinke als separates Bauteil am Drehmomentschlüssel fixiert sein. Der Drehmomentschlüssel ist zumindest im wesentlichen aus Edelstahl, Titan, Keramik oder Kunststoff hergestellt. Für den Herstellungsprozess eignen sich das Laser- und Wasserstrahlschneiden, Drahterodieren, Fräsen, Stanzen, Spritzgiessen und Metalldruckguss besonders.

Als wesentliche Vorteile des erfindungsgemässen Drehmomentschlüssels sind hervorzuheben:
- die zumindest prinzipielle Einstückigkeit begünstigt die Reinigung des Instruments, so entfällt jedes Demontieren und erneutes oft mühsames Zusammensetzen;
- das Instrument erlaubt die reversible Betätigung im Ratschenmodus;
- das generierte Drehmoment lässt sich ablesen; und
- bei Auswahl eines geeigneten Werkstoffs und Bearbeitungsverfahrens wird die Serienproduktion zu effizienten Herstellungskosten ermöglicht.

### Kurzbeschreibung der beigefügten Zeichnungen

Es zeigen:

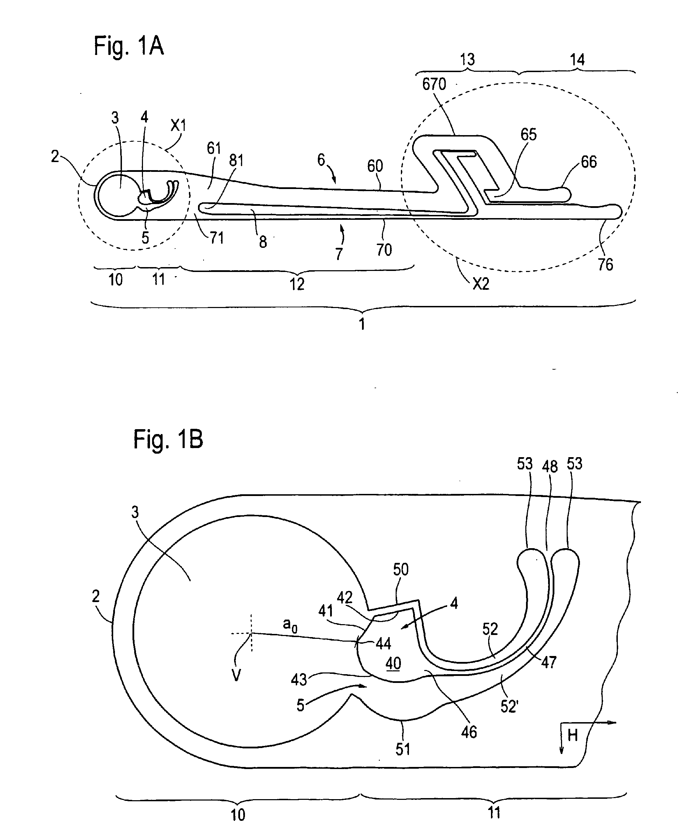

- Figur 1A:: einen erfindungsgemässen Drehmomentschlüssel *erster Variante,* in Draufsicht;
- Figur 1 B:: das vergrösserte Detail X1 aus Figur 1A;

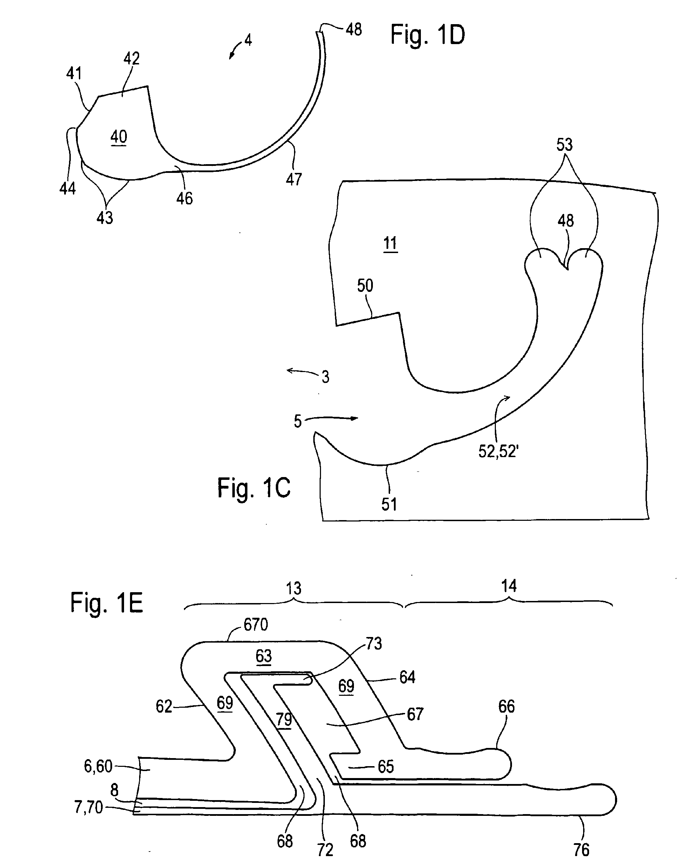

- Figur 1C:: den vergrösserten Halsbereich aus dem Detail X1 mit heraus getrennter Klinke;
- Figur 1 D:: eine aus Figur 1A heraus getrennte Klinke, in vergrösserter Darstellung;
- Figur 1E:: das vergrösserte Detail X2 aus Figur 1A;
- Figur 1F:: den Basisast aus dem Detail X2, in vergrösserter Darstellung;
- Figur 1 G:: den Biegeast aus dem Detail X2, in vergrösserter Darstel lung;
- Figur 2A:: ein Eindrehinstrument *erster Variante,* in Frontansicht;
- Figur 2B:: den Drehmomentschlüssel *erster Variante* gemäss Figur 1A mit dem angenäherten Eindrehinstrument gemäss Figur 2A, in Perspektivdarstellung;
- Figur 3A:: den Drehmomentschlüssel *erster Variante* gemäss Figur 1A mit eingesetztem Eindrehinstrument gemäss Figur 2A, in *Ruhestellung,* in Draufsicht;
- Figur 3B:: die Anordnung gemäss Figur 3A, in frontaler Perspektivdarstellung;
- Figur 3C:: die Anordnung gemäss Figur 3A, in der Perspektive von unten;
- Figur 3D:: das vergrösserte Detail X3 aus Figur 3A;
- Figur 4A:: den Drehmomentschlüssel *erster Variante* gemäss Figur 1A mit eingesetztem Eindrehinstrument gemäss Figur 2A, in *Vorwärtsrichtung* bis zur Maximalauslenkung des Biegeasts betätigt, in Draufsicht;
- Figur 4B:: das vergrösserte Detail X4 aus Figur 4A;
- Figur 4C:: das vergrösserte Detail X5 aus Figur 4A;
- Figur 5A:: den Drehmomentschlüssel *erster Variante* gemäss Figur 1A mit eingesetztem Eindrehinstrument gemäss Figur 2A, in *Rückwärtsrichtung* betätigt, in Draufsicht;
- Figur 5B:: das vergrösserte Detail X6 aus Figur 5A, ohne Eindrehinstrument;
- Figur 5C:: das vergrösserte Detail X6 aus Figur 5A;

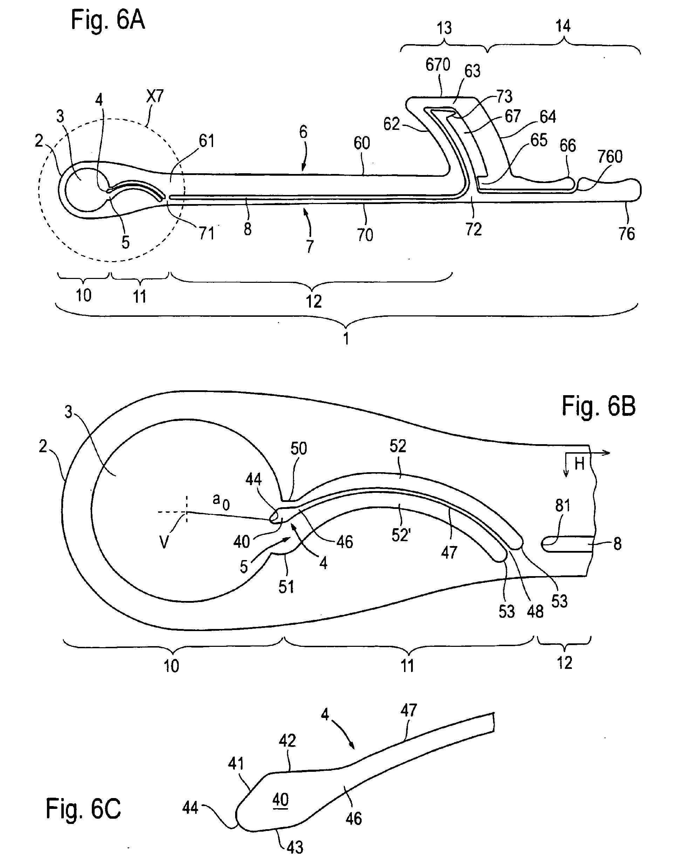

- Figur 6A:: einen erfindungsgemässen Drehmomentschlüssel *zweiter Variante,* in Draufsicht;
- Figur 6B:: das vergrösserte Detail X7 aus Figur 6A;
- Figur 6C:: eine aus Figur 6A heraus getrennte Klinke, in Vergrösserung;

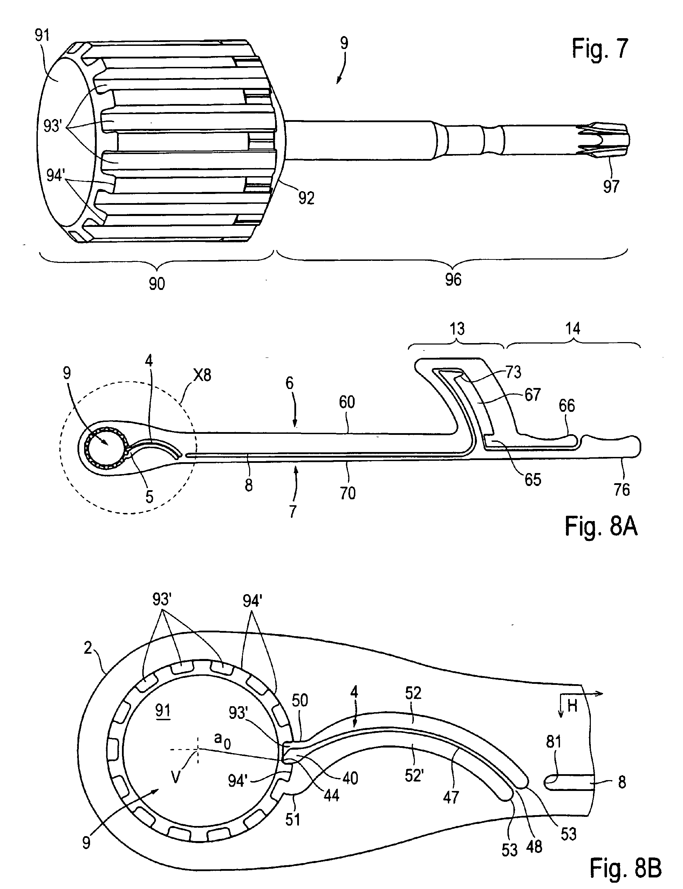

- Figur 7:: ein Eindrehinstrument *zweiter Variante,* in Frontansicht;
- Figur 8A:: den Drehmomentschlüssel *zweiter Variante* gemäss Figur 6A mit eingesetztem Eindrehinstrument *zweiter Variante* gemäss Figur 7, in *Ruhestellung,* in Draufsicht;
- Figur 8B:: das vergrösserte Detail X8 aus Figur 8A;
- Figur 9A:: den Drehmomentschlüssel *zweiter Variante* gemäss Figur 6A mit eingesetztem Eindrehinstrument *zweiter Variante* gemäss Figur 7, in *Vorwärtsrichtung* bis nahe zur Maximalauslenkung des Biegeasts betätigt, in Draufsicht;
- Figur 9B:: das vergrösserte Detail X9 aus Figur 9A;

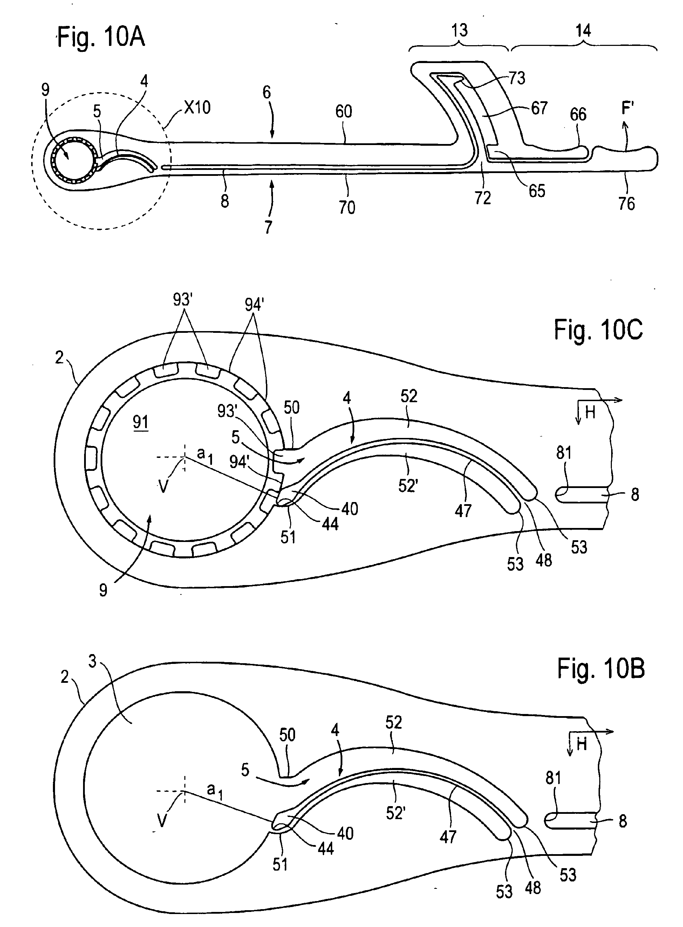

- Figur 10A:: den Drehmomentschlüssel *zweiter Variante* gemäss Figur 6A mit eingesetztem Eindrehinstrument *zweiter Variante* gemäss Figur 7, in *Rückwärtsrichtung* betätigt, in Draufsicht;
- Figur 10B:: das vergrösserte Detail X10 aus Figur 10A, ohne Eindrehinstrument;
- Figur 10C:: das vergrösserte Detail X10 aus Figur 5A;

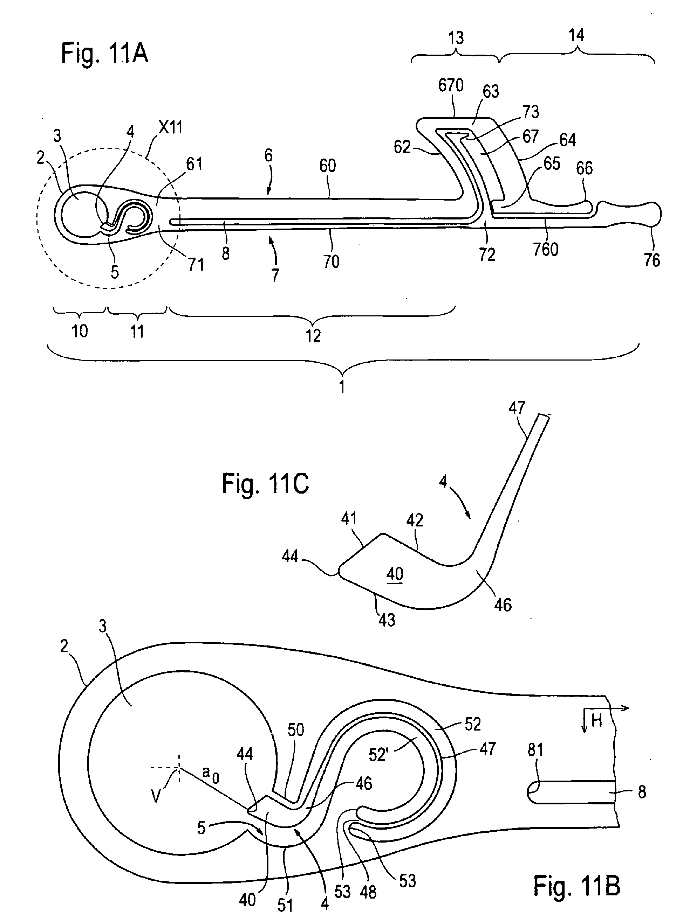

- Figur 11A:: einen erfindungsgemässen Drehmomentschlüssel *dritter Variante,* in Draufsicht;
- Figur 11B:: das vergrösserte Detail X11 aus Figur 11A;
- Figur 11C:: eine aus Figur 11A heraus getrennte Klinke, in Vergrösserung;

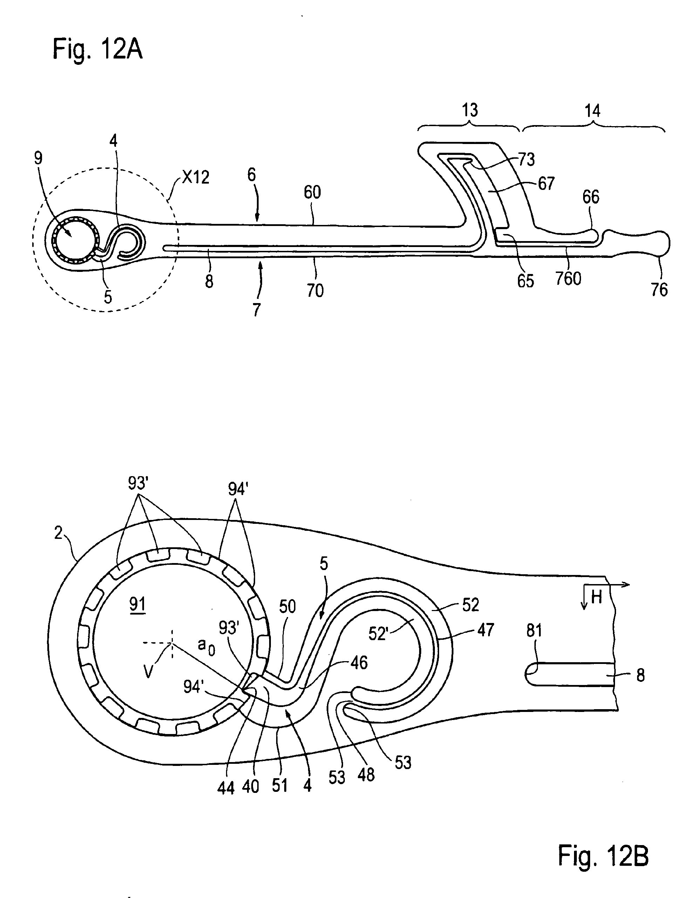

- Figur 12A:: den Drehmomentschlüssel *dritter Variante* gemäss Figur 11A mit eingesetztem Eindrehinstrument *zweiter Variante* gemäss Figur 7, in *Ruhestellung,* in Draufsicht;
- Figur 12B:: das vergrösserte Detail X12 aus Figur 12A;

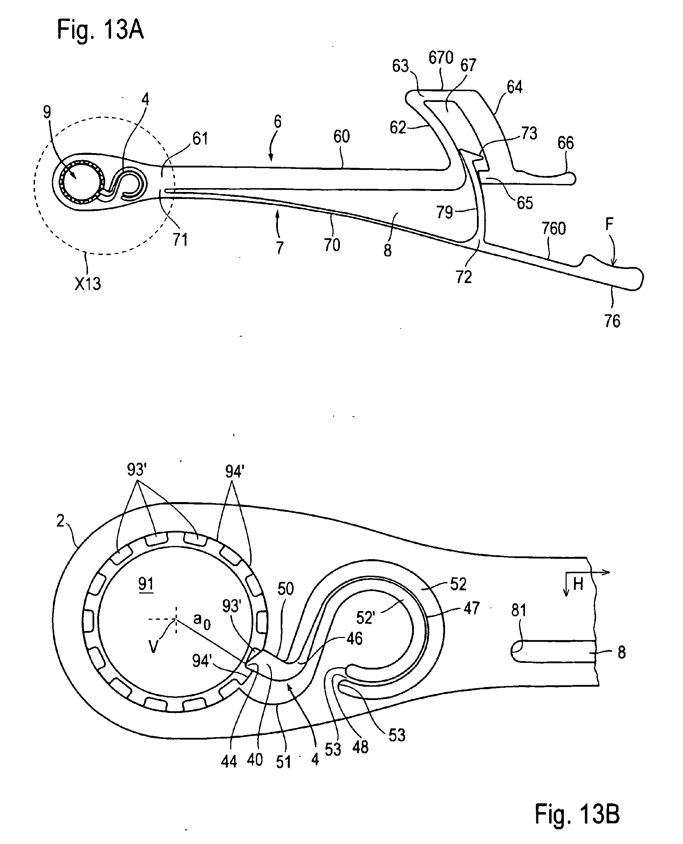

- Figur 13A:: den Drehmomentschlüssel *dritter Variante* gemäss Figur 11A mit eingesetztem Eindrehinstrument *zweiter Variante* gemäss Figur 7, in *Vorwärtsrichtung* bis nahe zur Maximalauslenkung des Biegeasts betätigt, in Draufsicht;
- Figur 13B:: das vergrösserte Detail X13 aus Figur 13A;

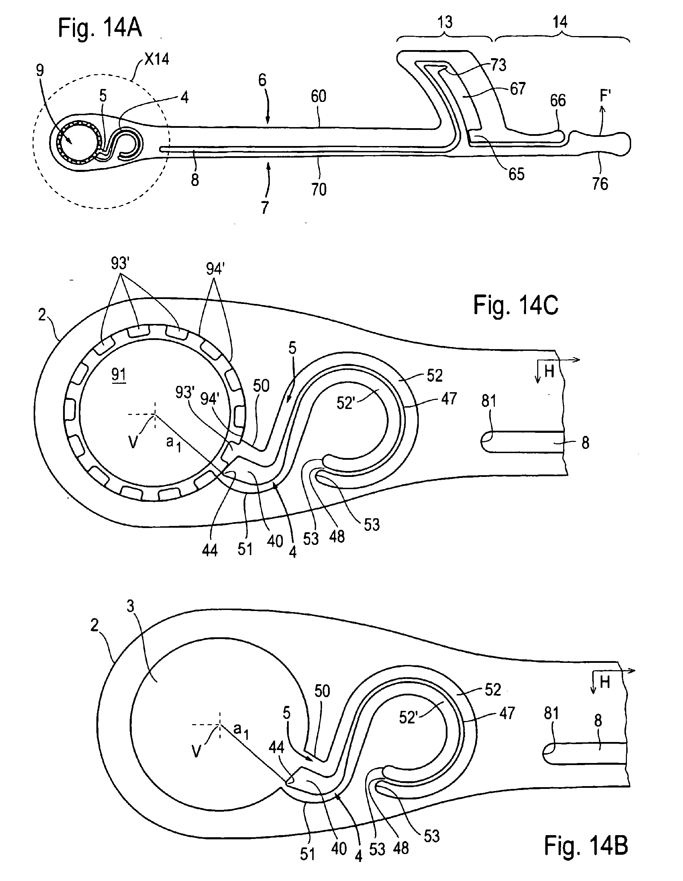

- Figur 14A:: den Drehmomentschlüssel *dritter Variante* gemäss Figur 11A mit eingesetztem Eindrehinstrument *zweiter Variante* gemäss Figur 7, in *Rückwärtsrichtung* betätigt, in Draufsicht;
- Figur 14B:: das vergrösserte Detail X14 aus Figur 14A, ohne Eindrehinstrument; und
- Figur 14C:: das vergrösserte Detail X14 aus Figur 14A.

### Ausführungsbeispiel

Anhand der beiliegenden Zeichnungen erfolgt nachstehend die detaillierte Beschreibung eines Ausführungsbeispiels zum erfindungsgemässen Drehmomentschlüssel.

Für die gesamte weitere Beschreibung gilt folgende Festlegung: Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden Figurenbeschreibungen Bezug genommen. Im Interesse der Übersichtlichkeit wird auf die wiederholte Bezeichnung von Bauteilen in nachfolgenden Figuren zumeist verzichtet, sofern zeichnerisch eindeutig erkennbar ist, dass es sich um "wiederkehrende" Bauteile handelt.

### Figuren 1A bis 1G

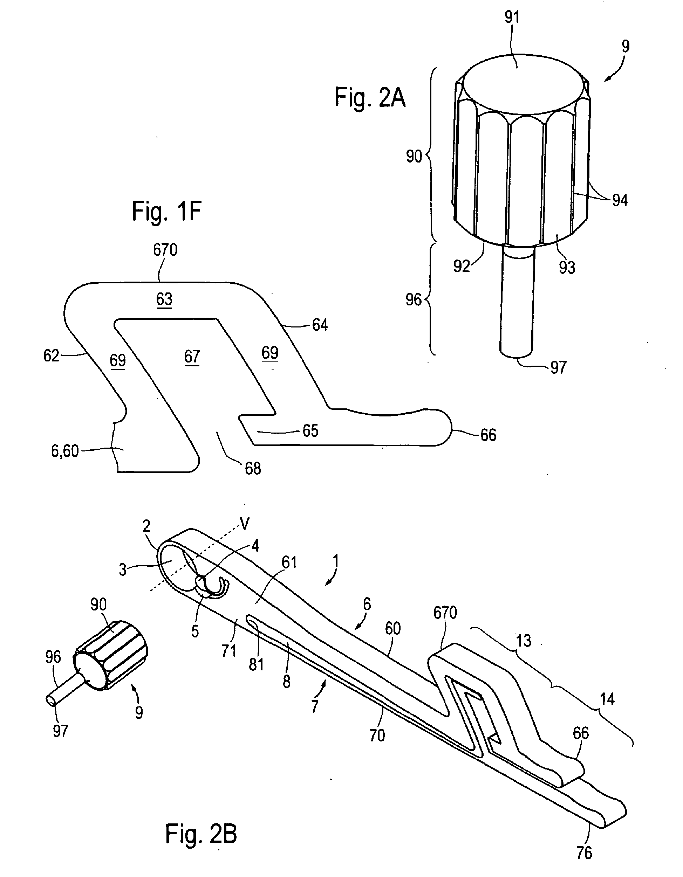

In dieser ersten Figurenfolge wird der konstruktive Aufbau des Drehmomentschlüssels 1 *erster Variante* ohne ein darin aufgenommenes Eindrehinstrument **9** (s. Figuren 2A und 2B) beschrieben. Der Drehmomentschlüssel **1,** welcher vorteilhaft aus einem länglichen, flachen Materialstück besteht, weist zunächst einen Kopfbereich **10** auf, an den sich nacheinander ein Halsbereich **11,** ein Schaftbereich **12,** ein Indikatorbereich **13** und zuletzt ein Griffbereich **14** anschliessen. Die Erstreckungsrichtung des Drehmomentschlüssels **1** liegt damit im wesentlichen in der Ebene **H.** Der Kopfbereich **10** wird von der kreisförmigen Umfassung **2** gebildet, die eine nahezu vollkreisförmige Aufnahmeöffnung **3** umschliesst und von zwei Seiten in den Halsbereich **11** einmündet. Durch das Zentrum der Aufnahmeöffnung **3** und senkrecht zur Ebene **H** verläuft die Achse **V.** Als Material kommen insbesondere Edelstahl, Titan, Keramik oder Kunststoffe in Betracht, und für die Herstellung bevorzugt Laser- und Wasserstrahlschneiden, Drahterodieren, Fräsen, Stanzen, das Spritzgussverfahren oder der Metalldruckguss.

Die Aufnahmeöffnung **3** ist in ihrem Durchmesser so bemessen, dass darin der Kopf **90** eines Eindrehinstruments **9** schlupffrei Platz findet. Am Übergang vom Kopfbereich **10** zum Halsbereich **11** mündet ein Spielraum **5** peripher in die Aufnahmeöffnung **3** ein, der sich hier bogenförmig in den Halsbereich **11** hinein erstreckt, wobei die Erstreckung des Spielraums **5** alternativ abschnittsweise oder gänzlich gerade verlaufen kann. Im Spielraum **5** ist die Klinke **4** angeordnet, welche begrenzt in der Ebene **H** elastisch beweglich ist und von einer gekrümmten, blattförmigen Klinkenfeder **47** gehalten wird, die einerseits an einem Federabgang **46** in das Klinkensegment **40** und andererseits an einer Federmündung **48** in das Material des Halsbereichs **11** übergeht. Komplementär zum alternativ gerade gestaltbaren Spielraum **5** wäre die Klinkenfeder **47** dann ebenfalls gerade. An der peripheren Mündung in die Aufnahmeöffnung **3** wird der hier erweiterte Spielraum **5** von einem oberen ersten Anschlag **50 -** in beispielhafter Gestalt einer schrägen Kante - und einem dazu gegenüber liegenden unteren zweiten Anschlag **51 -** in beispielhafter Gestalt einer Mulde **51** von vorzugsweise rundlicher Form - begrenzt. Von den Anschlägen **50,51** setzt sich der Spielraum **5** als im Bogen geführter, kanalförmiger oberer und unterer Federfreiraum **52,52'** bis zum Boden **53** fort, wo die Federmündung **48** ansetzt. Die Klinkenfeder **47** durchragt den Federfreiraum **52,52'** im Prinzip mittig, so dass beidseits der Klinkenfeder **47** als Federfreiraum **52,52'** ein Luftspalt verbleibt, welcher deren Beweglichkeit erlaubt.

In der unausgelenkten *Ruhestellung* befindet sich das Klinkensegment **40** mit seiner oberen zweiten Flanke **42** nahe des ersten Anschlags **50,** so dass ein schmaler Bewegungsspalt verbleibt. Die untere dritte Flanke **43** des Klinkensegments **40** ist nahezu maximal vom unteren zweiten Anschlag **51** entfernt. An die zweite Flanke **42** schliesst sich in Richtung der Aufnahmeöffnung **3** eine konkave erste Flanke **41** an, die sich bis zu einer Frontnase **44** erstreckt, welche jetzt zur Achse **V** den Minimalabstand **a₀** einnimmt. Die Frontnase **44** ist z.B. als in Richtung der Aufnahmeöffnung **3** erhabene Spitze oder Auswölbung gestaltet. Unterhalb der Frontnase **44** folgt die dritte Flanke **43 -** hier z.B. als Abrundung -, die sich bis zum Federabgang **46** fortsetzt. Der auf der Seite des zweiten Anschlags **51** liegende zweite Federfreiraum **52',** ist im Bereich des zweiten Anschlags **51** und daran angrenzend, momentan breiter als der erste Federfreiraum **52,** welcher an den ersten Anschlag **50** angrenzt.

Aus dem Halsbereich **11** heraus erstrecken sich der breitere, steife Basisast **6** sowie der schmale, federnde Biegeast **7,** beginnend an den Übergängen **61,71,** über den Schaftbereich **12** jeweils als Längsschenkel **60,70,** hin zum Indikatorbereich **13.** Zwischen dem Basisast **6** und dem Biegeast **7** liegt ein Luftspalt als Freischnitt **8,** welcher die elastische Auslenkung des Biegeasts **7** unabhängig vom Basisast **6** erlaubt. Dieser Freischnitt **8** verläuft von einem Kehlgrund **81,** der benachbart zu den Übergängen **61,71** vorgesehen ist, bis in den Indikatorbereich **13** hinein. Im Indikatorbereich **13** setzt sich der Längsschenkel **60** mit einem mäanderförmig verlaufenden ersten, zweiten und dritten Basisabzweig **62,63,64** fort, die zusammen einen nach oben weisenden Bügel **670** ergeben und einen Freiraum **67** umschliessen. Der dritte Basisabzweig **64** trifft L-förmig auf den frei nach aussen endenden Basisgriff **66,** an den sich der nach innen, in Richtung des Längsschenkels **60** weisende Anschlagschenkel **65** anschliesst. Zwischen dem Anschlagschenkel **65** und der Verbindung aus Längsschenkel **60** und erstem Basisabzweig **62** verbleibt ein zum Freiraum **67** führender Durchgang **68.**

Unterhalb des Durchgangs **68** setzt an den Längsschenkel **70** des Biegeasts 7 ein erster Abzweig **72** an, der sich im Freiraum **67** parallel zum ersten Basisabzweig **62** auf den zweiten Basisabzweig **63** zu erstreckt. L-förmig schliesst sich an den ersten Abzweig **72** ein zweiter Abzweig **73** an, der im Freiraum **67** parallel zum zweiten Basisabzweig **63,** bis nahe an den dritten Basisabzweig **64** verläuft. Hinter dem Ansatz des ersten Abzweigs **72** geht der Längsschenkel **70** des Biegeasts **7** in ein Griffteil **76** über, welches sich in der axialen Verlängerung des Längsschenkels **70** über den Basisgriff **66** hinaus erstreckt.

Zur Sichtbarmachung der auf den Biegeast **7** ausgeübten Auslenkung und des damit auf das Eindrehinstrument **9** übertragenen Drehmoments ist zumindest auf einem der Basisabzweige **62,64** eine erste Messmarkierung **69** angebracht, während der erste oder zweite Abzweig **72,73** des Biegeasts **7** eine komplementäre zweite Messmarkierung **79** aufweist. Die erste Messmarkierung **69** könnte eine Massskala sein, z.B. mit dem Bereich 0 Ncm bis 40 Ncm, so dass für die zweite Messmarkierung **79** ein Zeigerelement in Gestalt eines Bezugsstrichs oder Pfeils genügt, das bei Auslenkung das generierte Drehmoment an der Skala anzeigt.

### Figuren 2A und 2B

Dieses Figurenpaar zeigt das mit seinem Kopf **90** in die Aufnahmeöffnung **3** einzusetzende herkömmliche Eindrehinstrument **9** in einer *ersten Variante.* Der im Prinzip zylindrische Kopf **90** besitzt die Oberseite **91** und die Unterseite **92.** Die zwischen Oberseite **91** und Unterseite **92** liegende Mantelfläche weist zum Erfassen durch den Anwender und für den Eingriff von Werkzeugen eine übliche Konturierung auf, die von vertikalen Nuten **93,** welche vorzugsweise von rundlicher Querschnittsform sind, und dazwischen liegenden vertikalen Stegen **94** gebildet wird. Von der Unterseite **92** erstreckt sich ein Schaft **96,** der an der Instrumentenspitze **97** endet und hier ein standardisiertes Profil zum Zusammenwirken mit der zu handhabenden Schraube aufweist.

### Figuren 3A bis 3D

Bei in die Aufnahmeöffnung **3** des Drehmomentschlüssels **1** eingesetztem Eindrehinstrument **9** steht letzteres mit seinem Kopf **90** und Schaft **96** in der Ausrichtung der Achse **V,** welche senkrecht die Ebene **H** durchragt. Der zylindrische Kopf **90** mit seiner äusseren Profilierung, die sich aus den Nuten **93** und den dazwischen liegenden Stegen **94** zusammensetzt, wird von der Umfassung **2** des Kopfbereichs **10** umschlossen. In der *Ruhestellung* befindet sich das Klinkensegment **40** mit seiner zweiten Flanke **42** nahe dem ersten Anschlag **50** und die Frontnase **44** nimmt zur Vertikalachse **V** den Minimalabstand **a₀** ein, wobei die an die Frontnase **44** angrenzende zweite Flanke **42** und ein Abschnitt der dritten Flanke **43** in einer zugewandten Nut **93** zu liegen kommen, so dass die Vorderfront des Klinkensegments **40** partiell von dem an die Nut **93** angrenzenden Steg **94** unterfasst wird. Der zweite Federfreiraum **52'** hat in dem an den zweiten Anschlag **51** angrenzenden Bereich momentan seine maximale Breite.

### Figuren 4A bis 4C

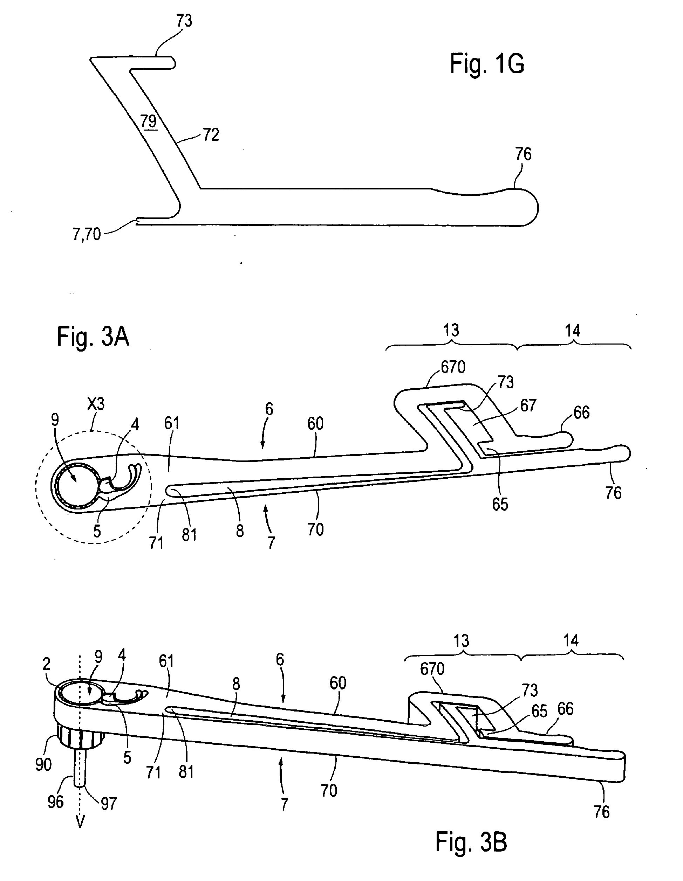

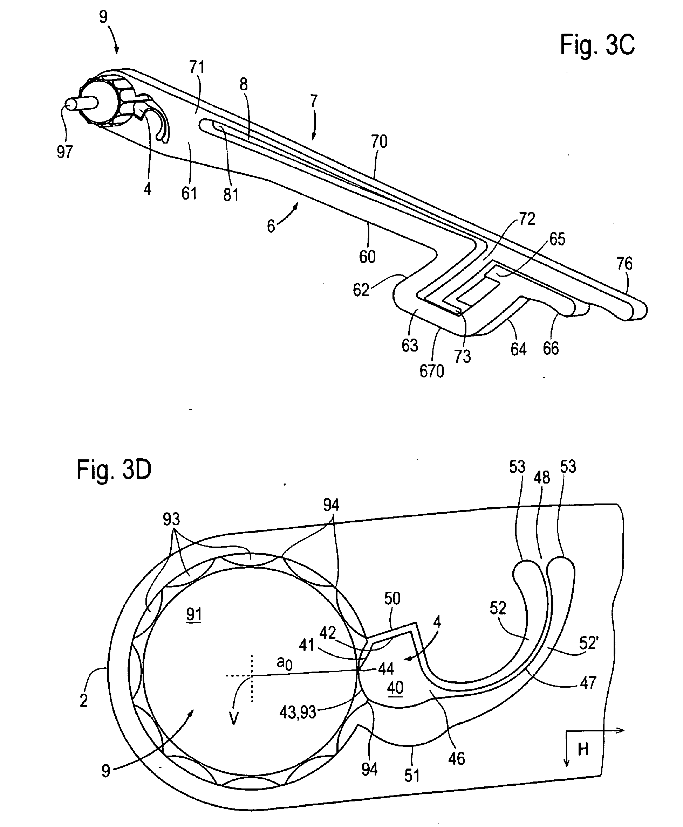

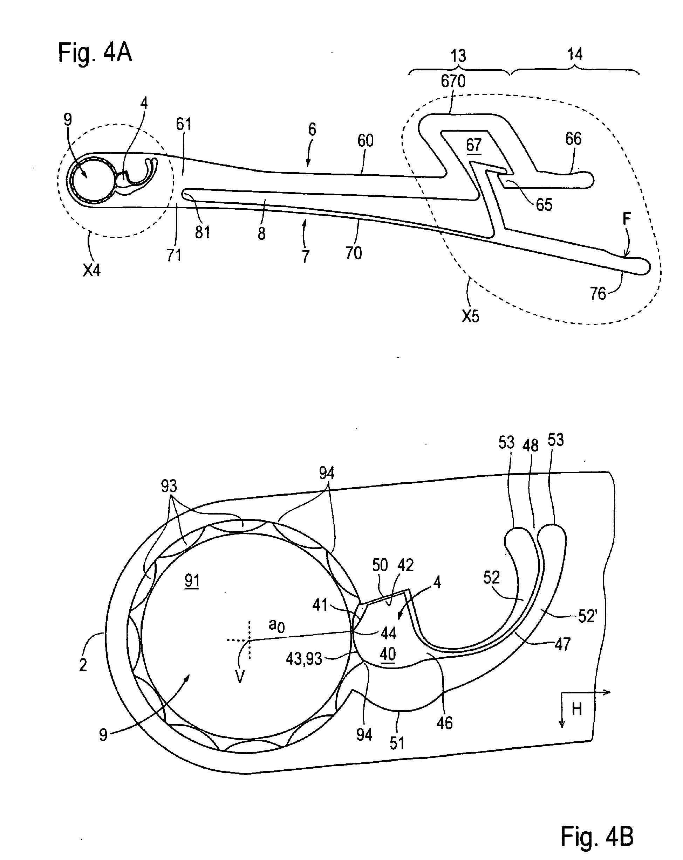

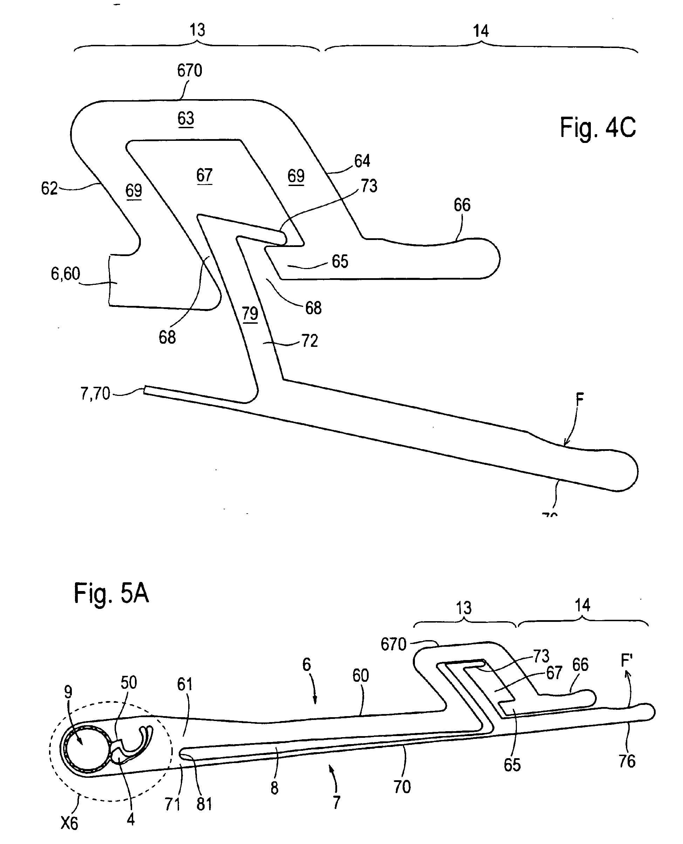

Wird auf den Biegeast **7** mit seinem Griffteil **76** eine Vorwärtskraft **F** ausgeübt, hat dies eine elastische Deformation mit Aufweitung des Freischnitts **8** im Indikatorbereich **13** zur Folge, die sich verringernd bis in den Kehlgrund **81** erstreckt. Zur Auslenkung des Biegeasts **7** wird der Anwender das Griffteil **76** erfassen und im Uhrzeigersinn - d.h. in einschraubender *Vorwärtsrichtung,* weg vom Basisgriff **66 -** mit der Vorwärtskraft **F** bewegen, bis an den Messmarkierungen **69,79** das vorgeschriebene Drehmoment erreicht ist. Zum Schutz vor Überdehnung des Biegeasts **7** gerät der zweite Abzweig **73** bei Maximalauslenkung des Biegeasts **7** an den Anschlagschenkel **65,** so dass ein weiteres Aufbiegen blockiert ist. Durch den formschlüssigen Eingriff zwischen der Frontnase **44** und der angrenzenden dritten Flanke **43** mit der zugewendeten Nut **93** und dem angrenzenden Steg **94** wird bei rechtsdrehender Einschraubbewegung des Drehmomentschlüssels **1** das Eindrehinstrument **9** mit in Rotation versetzt. Als Folge des beim Einschrauben auftretenden Widerstands wird die zweite Flanke **42** gegen die Elastizität der Klinkenfeder **47** an den ersten Anschlag **50** gedrückt, so dass der zuvor noch vorhandene Luftspalt geschlossen ist. Infolge der geringen Lageveränderung des Klinkensegments **40** kann man von einen im Prinzip unveränderten Minimalabstand **a₀** zwischen der Vertikalachse **V** und der Frontnase **44** ausgehen. Insoweit besteht dennoch ein Unterschied zwischen den Situationen gemäss Figur 3D (*Ruhestellung*) und Figur 4B (Belastung in einschraubender *Vorwärtsrichtung*).

### Figuren 5A bis 5C

Je nach Raumverhältnissen, z.B. in der Enge eines Patientenmundes, wurde der Drehmomentschlüssel **1** gemäss der Figur 4A in einschraubender *Vorwärtsrichtung* über ein mögliches Bogenmass betätigt, worauf bei erforderlichem weiteren Einschrauben der Drehmomentschlüssel **1** nun in einem reversiblen, entgegen dem Uhrzeigersinn gerichteten Takt - in seiner Funktion als Ratscheninstrument - in *Rückwärtsrichtung* mit Rotationsbewegung um die Achse **V** mit der Rückwärtskraft **F'** zu führen ist.

Bei der *Rückwärtsbewegung* verharrt das Eindrehinstrument **9** infolge des auf die applizierte Schraube wirkenden Widerstands in seiner Position. Zugleich wird das Klinkensegment **40** gegen die elastische Wirkung der Klinkenfeder **47** vom ersten Anschlag **50** hin zum zweiten Anschlag **51** geführt, so dass sich zwischen der Achse **V** und der Frontnase **44** der Maximalabstand **a₁** einstellt.

Der erreichte Maximalabstand **a₁** erlaubt ein Vorbeifahren des Stegs **94** an der ersten Flanke **41** und der Frontnase **44.** Auf diese Weise können bei der *Rückwärtsbewegung* des Drehmomentschlüssels **1** mehrere Stege **94** vom Klinkensegment **40** umfahren werden. Beim erneuten Wechsel in die *Vorwärtsbewegung* entfällt die Kraft, welche das Klinkensegment **40** in Richtung zweiten Anschlag **51** drückt, wodurch das Klinkensegment **40** von der Klinkenfeder **47** an den ersten Anschlag **50** rückgeführt wird und die Situation für das Einschrauben gemäss Figur 4A wieder hergestellt ist.

### Figuren 6A bis 6C

Diese Figurengruppe zeigt den Drehmomentschlüssel **1** in einer *zweiten Variante,* der sich wiederum in den Kopfbereich **10,** den Halsbereich **11,** den Schaftbereich **12,** den Indikatorbereich **13** und den Griffbereich **14** strukturiert. Der wesentliche Unterschied zur Vorgängervariante besteht in der Gestalt der Klinke **4** und des diese umgebenden Spielraums **5.** Im Verhältnis zur Unterkante des Drehmomentschlüssels **1** verlaufen Klinke **4** und Spielraum **5** jetzt in konvexer Form, das Klinkensegment **40** hat einen eher zungenförmigen Zuschnitt und der obere erste Anschlag **50** wird von einer kürzeren im Prinzip geraden Kante gebildet. Der Kopfbereich **10** besitzt die kreisförmige Umfassung **2,** welche die im Prinzip einen Vollkreis darstellende Aufnahmeöffnung **3** umgibt, in deren Zentrum senkrecht zur Ebene **H** die Achse **V** verläuft. Gegenüber dem ersten Anschlag **50** liegt unverändert unten an der Mündung des Spielraums **5** der muldenartige zweite Anschlag **51.** Die Klinkenfeder **47** erstreckt sich vom Klinkensegment **40** ab dem Federabgang **46** bis zur Federmündung **48** und geht dort in den Halsbereich **11** über. Beidseits der Klinkenfeder **47** verlaufen der erste und zweite Federfreiraum **52,52',** welche einerseits am Boden **53** jeweils neben der Federmündung **48** enden und andererseits neben dem Klinkensegment **40** in die Aufnahmeöffnung **3** eintreten. Das Klinkensegment **40** endet vorn mit der in die Aufnahmeöffnung **3** hineinragenden Frontnase **44,** von der sich nach oben eine erste Flanke **41** erstreckt, die in eine zweite Flanke **42** übergeht, welche auf den Federabgang **46** zuläuft. Nach unten geht die Frontnase **44** in eine dritte Flanke **43** über, die sich zum Federabgang **46** hin erstreckt. In Ruhestellung ergibt sich zwischen der Achse **V** und der Frontnase **44** der Minimalabstand **a₀.** Von den Übergängen **61,71** am Ende des Halsbereichs **11** erstrecken sich der Basisast **6** und der Biegeast **7** zunächst mit ihren Längsschenkeln **60,70,** zwischen denen der Freischnitt **8** liegt, welcher zum Kopfbereich **10** hin im Kehlgrund **81** endet. Das Griffteil **76** hat auf seiner Oberseite eine lang gestreckte Vertiefung **760,** in welcher der Basisgriff **66** Platz findet. Die übrigen Merkmale sind gegenüber der *ersten Variante* des Drehmomentschlüssel **1** im Prinzip unverändert; dazu gehören auch die Messmarkierungen **69,79.**

### Figur 7

Bei der *zweiten Variante* des Eindrehinstruments **9** sind wiederum der Kopf **90** mit seiner Oberseite **91** und Unterseite **92** vorhanden, wobei sich zentrisch von letzterer der Schaft **96** bis zur Instrumentenspitze **97** erstreckt. Im Unterschied zur *ersten Variante* des Eindrehinstruments **9** haben die Nuten **93'** nun einen im Prinzip eckigen Querschnitt und die dazwischen liegenden Stege **94'** sind ebenfalls eckig, so dass sich zirkulär eine Mäanderform ergibt.

### Figuren 8A und 8B

Ist das Eindrehinstrument **9** in die Aufnahmeöffnung **3** des Drehmomentschlüssels **1** eingesetzt, stehen Kopf **90** und Schaft **96** in Ausrichtung der Achse **V.** Der zylindrische Kopf **90** mit der aus den Nuten **93'** und Stegen **94'** bestehenden Profilierung ist von der Umfassung **2** umschlossen. In *Ruhestellung -* das Griffteil **76** bleibt unbetätigt, wird also weder im Uhrzeigersinn gezogen noch rückläufig bewegt - ist die gesamte Klinke **4** nicht ausgelenkt, die Klinkenfeder **47** somit spannungsfrei und die beiden Federfreiräume **52,52'** erstrecken sich in unverändertem Verlauf. Das Klinkensegment **40** steht im geringen Abstand zum ersten und zweiten Anschlag **50,51** und ragt in die nächstliegende Nut **93'** hinein. Zwischen der Achse **V** und der Frontnase **44** existiert der Minimalabstand **a₀**.

### Figuren 9A und 9B

Dieses Figurenpaar korrespondiert im Prinzip mit den Figuren 4A bis 4C und zeigt den Drehmomentschlüssel **1** *zweiter Variante* mit eingesetztem Eindrehinstrument **9** in *Vorwärtsrichtung* bewegt, d.h. auf das Griffteil **76** wirkt im Uhrzeigersinn eine Kraft **F** ein, wodurch der Biegeast **7** bis nahe an die Maximalauslenkung verformt ist. Der erste Abzweig **72** ist zum grössten Teil aus dem Freiraum **67** herausgefahren und der als Anschlag dienende zweite Abzweig **73** steht nahe dem Anschlagschenkel **65.** Zugleich ist die Vertiefung **760** völlig offen, da sich der Basisgriff **66** nicht mehr darin befindet. Auf das z.B. in eine Schraube eingreifende Eindrehinstrument **9** wird durch das zu überwindende Anzugsmoment eine Widerstandskraft ausgeübt, so dass - je nach Betrag des Anzugsmoments - das Klinkensegment **40** vom benachbarten Steg **94'** in Richtung des ersten Anschlags **50** ausgelenkt wird. Die Frontnase **44** sitzt mit maximaler Eingriffstiefe in einer Nut **93',** wobei die Distanz zwischen der Frontnase **44** und der Achse V im Prinzip unverändert bleibt und weiterhin den Minimalabstand **a₀** einnimmt. Bei Maximalauslenkung steht das Klinkensegment **40** am ersten Anschlag **50** an. In Folge der Auslenkung der Klinke **4** hat sich die Relation zwischen den Federfreiräumen **52,52'** verändert, indem der erste Federfreiraum **52** nun verengt und der zweite Federfreiraum **52'** entsprechend erweitert ist. Solange von der auf das Griffteil **76** einwirkenden Kraft **F** das Anzugsmoment überwunden wird, bewegt sich der Drehmomentschlüssel **1** im Uhrzeigersinn und nimmt dabei das darin eingesetzte Eindrehinstrument **9** mit.

### Figuren 10A bis 10C

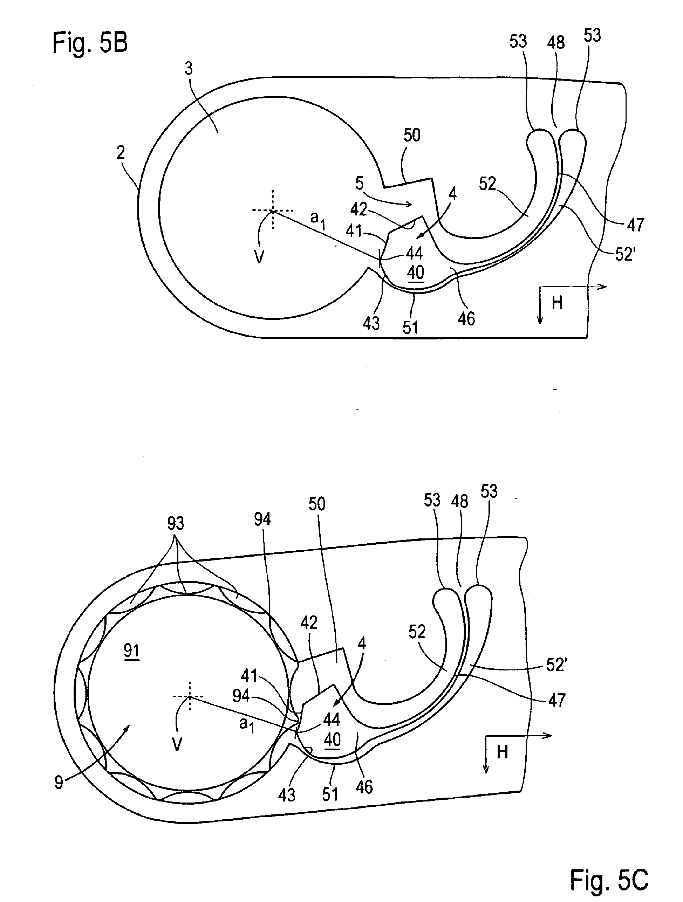

Diese Figurengruppe korrespondiert im Prinzip mit den Figuren 5A bis 5C und zeigt den Drehmomentschlüssel ***1** zweiter Variante* mit eingesetztem Eindrehinstrument **9** in *Rückwärtsrichtung* bewegt, d.h. auf das Griffteil **76** wirkt eine dem Uhrzeigersinn entgegen gesetzte Kraft **F'** ein. Der Basisast **6** und der Biegeast **7** liegen nahe beieinander, letzterer und der Freischnitt **8** sind unverformt. Der erste Abzweig **72** und der zweite Abzweig **73** sind maximal in den Freiraum **67** eingefahren. Das nicht überwundene Lösemoment bewirkt ein Stehenbleiben des Eindrehinstruments **9,** wodurch der zum Klinkensegment **40** nächststehende Steg **94'** die Auslenkung der Klinke **4** bewirkt. Folglich wird das Klinkensegment **40** in Richtung des zweiten Anschlags **51** gedrückt und zwischen dem Klinkensegment **40** sowie der Achse **V** stellt sich der Maximalabstand **a₁** ein, so dass das Klinkensegment **40 -** abgesehen von der Berührung der zirkulären Oberfläche der Stege **94' -** ausser Eingriff mit dem Kopf **90** des Eindrehinstruments **9** kommt. Durch Verbiegen der Klinkenfeder **47** ist nun der erste Federfreiraum **52** erweitert, während sich der zweite Federfreiraum **52'** verengt hat.

### Figuren 11A bis 12B.

Jetzt liegt der Drehmomentschlüssel **1** in einer *dritten Variante* vor. Modifiziert sind lediglich die gesamte Klinke **4** mit dem Klinkensegment **40** und der Verlauf der Klinkenfeder **47** sowie der Spielraum **5** mit dem angrenzenden oberen ersten Anschlag **50** und dem unteren, muldenförmigen zweiten Anschlag **51.** Das Klinkensegment **40** hat nun eher eine eckige Zungenform, endet vorn mit der in die Aufnahmeöffnung **3** hineinragenden Frontnase **44,** von der nach oben die erste Flanke **41** verläuft, an die sich eine zweite Flanke **42** anschliesst, welche auf den Federabgang **46** trifft. Nach unten folgt der Frontnase **44** eine dritte Flanke **43,** die in den Federabgang **46** übergeht. Der Spielraum **5** windet sich nun von der Mündung in die Aufnahmeöffnung **3** schneckenförmig innerhalb des Halsbereichs **11** hin zum Boden **53.** Die Klinkenfeder **47** durchläuft den Spielraum **5** ebenfalls schneckenförmig, teilt den Spielraum **5** wiederum in einen ersten und einen zweiten Federfreiraum **52,52'** und endet an der Federmündung **48.** Der erste Anschlag **50** ist steiler ausgebildet, und das Klinkensegment **40** steht in der momentanen *Ruhestellung* zur Achse **V** im Minimalabstand **a₀.** Die Muldenkontur des unteren Anschlags **51** ist ausgeprägter gestaltet. Alle übrigen Elemente am Drehmomentschlüssel **1** sind gegenüber den Vorgängervarianten unverändert, dazu gehören auch die Messmarkierungen **69,79.**

In den Drehmomentschlüssel **1** *dritter Variante* wird auch das Eindrehinstrument **9** *zweiter Variante* eingesetzt. In der *Ruhestellung -* auf das Griffteil **76** wirken weder eine Vorwärtskraft **F** noch eine Rückwärtskraft **F'** ein und der Basisgriff **66** liegt in der Vertiefung **760 -** ragt die Frontnase **44** maximal in eine Nut **93'** und die gesamte Klinke **4** mit dem Klinkensegment **40** und der davon abgehenden Klinkenfeder **47** sind innerhalb des Spielraums **5** nicht ausgelenkt. Das Klinkensegment **40** steht zu beiden Anschlägen **50,51** im Abstand.

### Figuren 13A und 13B

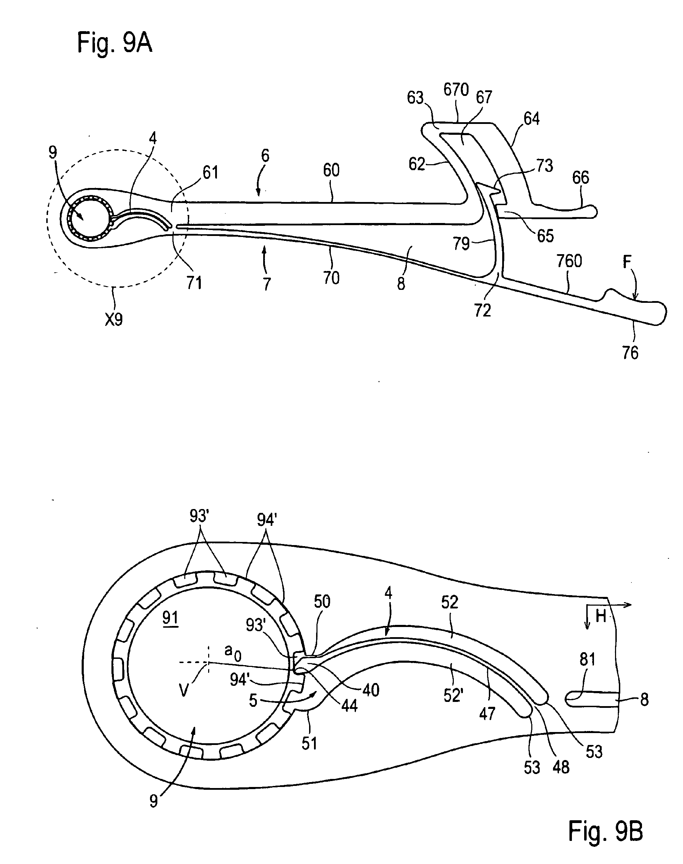

Bei Betätigung des Drehmomentschlüssels **1** *dritter Variante* mit eingesetztem Eindrehinstrument **9** *zweiter Variante* in *Vorwärtsrichtung -* auf das ausgelenkte Griffteil **76** wirkt eine Vorwärtskraft **F** ein - ergibt sich eine analoge Situation zu den Figuren 4A bis 4C sowie 9A und 9B. Lediglich die Stellung der Klinke **4** und deren Zusammenwirken mit dem Eindrehinstrument **9** sind aufgrund der Modifikationen an Klinke **4,** Spielraum **5** und den angrenzenden Konturen verschieden. Aufgrund des auf das Eindrehinstrument **9** wirkenden Widerstands wird das mit seiner Frontnase **44** in einer Nut **93'** liegende Klinkensegment **40** gegen den ersten Anschlag **50** gedrückt, wobei auch die Klinkenfeder **47** eine Auslenkung erfährt und dadurch sich die Relation zwischen beiden Federfreiräumen **52,52'** verschiebt. Der Minimalabstand **a₀** zwischen der Frontnase **44** und der Achse **V** bleibt im Prinzip erhalten.

### Figuren 14A bis 14C

Diese Figurengruppe veranschaulicht den Drehmomentschlüssel **1** *dritter Variante* mit eingesetztem Eindrehinstrument **9** *zweiter Variante* bei Betätigung in *Rückwärtsrichtung,* d.h. am Griffteil **76** setzt die Rückwärtskraft **F'** an. In Folge der gehemmten Stellung des Eindrehinstruments **9** wird die Frontnase **44** des Klinkensegments **40** aus der zuvor belegten Nut **93'** bis auf die zirkuläre Oberfläche der Stege **94'** herausbewegt und dabei die gesamte Klinke **4** ausgelenkt, wobei das Klinkensegment **40** zum zweiten Anschlag **51** hin fährt. Die Frontnase **44** gerät zur Achse **V** in den Maximalabstand **a₁,** und der zweite Federfreiraum **52'** verengt sich insbesondere im Bereich des zweiten Anschlags **51,** während sich der erste Federfreiraum **52** insbesondere im Bereich des ersten Anschlags **50** erweitert.

## Patentansprüche

1. Drehmomentschlüssel **(1)** als Ratscheninstrument für die Medizinaltechnik mit:
a) einem zuvorderst liegenden Kopfbereich **(10),** einem anschliessenden Halsbereich **(11),** dem ein Schaftbereich **(12)** folgt, und einem zuhinterst angeordneten Griffbereich **(14),** welche sich in der Ebene **(H)** erstrecken;
b) einer im Kopfbereich **(10)** vorgesehenen Aufnahmeöffnung **(3),** die:
ba) von einer Umfassung **(2)** umgeben ist;
bb) einen Mittelpunkt besitzt, durch den sich eine Achse **(V)** zumindest im Prinzip senkrecht erstreckt; und
bc) zum Einsetzen eines herkömmlichen Eindrehinstruments **(9)** in der Erstreckung der Achse **(V)** dient, welche zur Ebene (H) senkrecht steht;
c) einem an der Peripherie der Aufnahmeöffnung **(3)** angeordneten, begrenzt beweglichen Klinkensegment **(40),** dessen Frontpartie **(41-44)** zur Aufnahmeöffnung **(3)** weist, wobei die Frontpartie **(41-44)** dazu bestimmt ist:
ca) bei Betätigung des Drehmomentschlüssels **(1)** in *Vorwärtsrichtung,* also im Einschraubmodus, mit einer am Kopf **(90)** des Eindrehinstruments **(9)** vorhandenen Aussenkontur **(93,94;93',94')** in mitnehmenden Eingriff zu kommen;
cb) bei Betätigung des Drehmomentschlüssels **(1)** in *Rückwärtsrichtung,* also im Ratschenmodus, den mitnehmenden Eingriff mit der am Kopf **(90)** des Eindrehinstruments **(9)** vorhandenen Aussenkontur **(93,94;93',94')** zu lösen; und
d) einem vom Halsbereich **(11)** längs des Drehmomentschlüssels **(1)** verlaufenden, biegesteifen Basisast **(6);**
e) einem auslenkbaren, vorzugsweise linear-elastischen Biegeast **(7),** über den man bei Betätigung des Drehmomentschlüssels **(1)** in *Vorwärtsrichtung* das zu generierende Drehmoment mittels einer vom Benutzer ausgeübten Vorwärtskraft **(F)** einbringt;
f) sich vom Klinkensegment **(40)** eine Klinkenfeder **(47)** in den Halsbereich **(11)** hinein erstreckt, wobei das Klinkensegment **(40)** und die Klinkenfeder **(47)** eine einteilige oder aus mehreren Teilen zusammengesetzte Klinke **(4)** bilden;
g) Klinkensegment **(40)** und Klinkenfeder **(47)** in einem kanalförmigen Spielraum **(5)** angeordnet sind, welcher die Auslenkung beider in der Ebene **(H)** gegen die Kraft der Klinkenfeder **(47)** erlaubt; und
h) die Klinke **(4)** einstückig aus dem Halsbereich **(11)** herausgeformt oder als separates Bauteil daran befestigt ist,
**dadurch gekennzeichnet, dass**
i) das Klinkensegment **(40)** an seiner Frontpartie **(41-44)** eine Frontnase **(44)** aufweist, von der einerseits eine zweite Flanke **(42)** und andererseits eine dritte Flanke **(43)** liegen;
j) der Spielraum **(5)** mit einem ersten und einem zweiten Anschlag **(50,51)** zur Aufnahmeöffnung **(3)** münden;
k) der erste Anschlag **(50)** der zweiten Flanke **(42)** gegenüber angeordnet ist und bei Betätigung des Drehmomentschlüssels **(1)** in *Vorwärtsrichtung* die zweite Flanke **(42)** der Klinke **(4)** am ersten Anschlag **(50)** ansteht, wobei die Frontnase **(44)** zur Achse **(V)** zumindest im Prinzip im Minimalabstand **(a₀)** steht; und
l) der zweite Anschlag **(51)** zumindest anteilig der dritten Flanke **(43)** gegenüber angeordnet ist und bei Betätigung des Drehmomentschlüssels **(1)** in *Rückwärtsrichtung* die dritte Flanke **(43)** der ausgelenkten Klinke **(4)** sich dem zweiten Anschlag **(51)** angenähert hat oder daran ansteht, wobei die Frontnase **(44)** zur Achse **(V)** im Maximalabstand **(a₁)** steht, die zweite Flanke **(42)** vom ersten Anschlag **(50)** entfernt positioniert ist und die Klinkenfeder **(47)** gegen deren Rückstellkraft elastisch deformiert ist.

2. Drehmomentschlüssel **(1)** nach Anspruch 1, **dadurch gekennzeichnet, dass**
a) die Klinkenfeder **(47):**
aa) einerseits in einem Federabgang **(46)** an das Klinkensegment **(40)** und andererseits in einer Federmündung **(48)** in den Halsbereich **(11)** übergeht; und
ab) die Gestalt einer geraden oder zumindest partiell bogenförmigen Blattfeder hat;
b) an die Frontnase **(44)** des Klinkensegments **(40):**
ba) in einer Richtung eine erste Flanke **(41)** ansetzt und sich daran benachbart die zweite Flanke **(42)** anschliesst;
bb) in der anderen Richtung die dritte Flanke **(43)** ansetzt, welche sich abgerundet in Richtung des Federabgangs **(46)** erstreckt; wobei
bc) die Frontnase **(44)** als eine in Richtung der Aufnahmeöffnung **(3)** erhabene Spitze oder Auswölbung ausgebildet ist;
c) der erste Anschlag **(50)** zumindest im Prinzip als gerade Kante und der zweite Anschlag **(51)** muldenförmig beschaffen ist; und
d) die den Spielraum **(5)** durchragende Klinkenfeder **(47)** einen ersten und einen zweiten Federfreiraum **(52,52')** abteilt, deren Proportionen sich mit der Bewegung der Klinkenfeder **(47)** ändern und in einem Boden **(53)** enden, an den die Federmündung **(48)** ansetzt.

3. Drehmomentschlüssel **(1)** nach zumindest einem der Ansprüche 1 und 2, **dadurch gekennzeichnet, dass**
a) sich der Basisast **(6)** von einem im Halsbereich **(11)** gelegenen Übergang **(61)** zunächst als Längsschenkel **(60)** über den Schaftbereich **(12)** bis in einen Indikatorbereich **(13)** hinein erstreckt und im Griffbereich **(14)** mit einem Basisgriff **(66)** frei endet;
b) der Biegeast **(7)** von einem im Halsbereich **(11)** gelegenen Übergang **(71)** im Prinzip parallel zum Längsschenkel **(60)** zunächst als langgestreckter Längsschenkel **(70)** bis in den Indikatorbereich **(13)** hinein verläuft und im Griffbereich **(14)** mit einem Griffteil **(76)** frei endet, welches den Basisgriff **(66)** überragt;
c) der Biegeast **(7)** an seiner Oberseite, dem Basisast **(6)** zugewandt, eine Vertiefung **(760)** aufweisen kann, in welcher der Anschlagschenkel **(65)** und der Basisgriff **(66)** partiell eingebettet sind; und
d) zwischen dem Längsschenkel **(60)** des Basisasts **(6)** und dem Längsschenkel **(70)** des Biegeasts **(7)** ein Freischnitt **(8)** liegt, der sich von einem an die Übergänge **(61,71)** angrenzenden Kehlgrund **(81)** bis in den Indikatorbereich **(13)** hinein erstreckt.

4. Drehmomentschlüssel **(1)** nach zumindest einem der Ansprüche 1 bis 3, **dadurch gekennzeichnet, dass**
a) der Indikatorbereich **(13)** gebildet wird von:
aa) zumindest einer Abzweigung **(62,63,64)** des Basisasts **(6),** die zwischen dem Längsschenkel **(60)** und dem Basisgriff **(66)** liegt; und
ab) zumindest einer Abzweigung **(72,73)** des Biegeasts **(7),** die zwischen dessen Längsschenkel **(70)** und Griffteil **(76)** liegt; und
b) mit der Betätigung des Drehmomentschlüssels **(1)** in *Vorwärtsrichtung* und der Auslenkung des Biegeasts **(7)** die Relativlage zwischen der zumindest einen Abzweigung **(62,63,64)** des Basisasts **(6)** und der zumindest einen Abzweigung **(72,73)** des Biegeasts **(7),** ausgehend von der *Ruhestellung* mit einer *Nullposition,* eine inkrementale Änderung erfährt, welche ein Mass für das generierte Drehmoment ist.

5. Drehmomentschlüssel **(1)** nach Anspruch 4, **dadurch gekennzeichnet, dass** auf der zumindest einen Abzweigung **(62,63,64)** des Basisasts **(6)** und der zumindest einen Abzweigung **(72,73)** des Biegeasts **(7)** eine erste bzw. eine zweite Messmarkierung **(69,79)** angebracht sind, an welchen die Auslenkung des Biegeasts **(7)** infolge der einwirkenden Vorwärtskraft **(F)** als erzeugtes Drehmoment ablesbar ist.

6. Drehmomentschlüssel **(1)** nach Anspruch 4, **dadurch gekennzeichnet, dass** der Indikatorbereich **(13)** besteht aus:
a) einem vom Längsschenkel **(60)** sich zum Basisgriff **(66)** erstreckenden Bügel **(670),** welcher von der zumindest einen Abzweigung **(62,63,64)** gebildet wird und einen Freiraum **(67)** umschliesst, zu dem ein Durchgang **(68)** vorhanden ist; und
b) der zumindest einen Abzweigung **(72,73)** des Biegeasts **(7),** welche durch den Durchgang **(68)** in den Freiraum **(67)** hinein ragt.

7. Drehmomentschlüssel **(1)** nach Anspruch 4, **dadurch gekennzeichnet, dass**
a) sich der Bügel **(670)** mäanderartig aus dem vom Längsschenkel **(60)** L-förmig abbiegenden ersten Basisabzweig **(62)** und dem sich daran nacheinander anschliessenden zweiten Basisabzweig **(63)** und dritten Basisabzweig **(64),** der L-förmig in den Basisgriff **(66)** übergeht, zusammensetzt;
b) sich an den ersten Abzweig **(72)** L-förmig ein zweiter Abzweig **(73)** anschliesst;
c) ein Anschlagschenkel **(65)** sich vom Basisgriff **(66)** nach innen, in Richtung des Längsschenkels **(60)** erstreckt und den Durchgang **(68)** einerseits begrenzt; wobei
d) der Freiraum **(67),** der Anschlagschenkel **(65)** und der Durchgang **(68)** als quasi ortsfeste Elemente und der erste Abzweig **(72)** mit dem zweiten Abzweig **(73)** als mit dem Biegeast **(7)** auslenkbare Elemente so dimensioniert sind, dass die Maximalauslenkung des Biegeasts **(7)** durch das Aufsetzen des zweiten Abzweigs **(73)** am Anschlagschenkel **(65)** definiert ist.

8. Drehmomentschlüssel **(1)** nach Anspruch 4, **dadurch gekennzeichnet, dass**
aa) die erste Messmarkierung **(69)** in Gestalt einer Massskala, z.B. mit dem Bereich 0 Ncm bis 40 Ncm, zumindest auf einem der Basisabzweige **(62,64),** vorzugsweise auf dem zum Basisgriff **(66)** benachbarten Basisabzweig **(64),** vorgesehen ist; und
ab) die zweite Messmarkierung **(79)** in Gestalt eines Zeigerelements, z.B. als Bezugsstrich oder Pfeil, auf dem ersten oder zweiten Abzweig **(72,73)** des Biegeasts **(7),** vorzugsweise auf dem frei endenden zweiten Abzweig **(73)** angebracht ist; oder
ba) die erste Messmarkierung **(69)** in Gestalt eines Zeigerelements, z.B. als Bezugsstrich oder Pfeil, auf dem Anschlagschenkel **(65)** des Basisastes **(6),** in Richtung des ersten Abzweigers **(72)** des Biegeastes **(7)** angebracht ist; und
bb) die zweite Messmarkierung **(79)** in Gestalt einer Massskala, z.B. mit dem Bereich 0 Ncm bis 40 Ncm, auf dem 1. Abzweig **(72)** vorgesehen ist.

9. Drehmomentschlüssel **(1)** nach zumindest einem der Ansprüche 1 bis 8, **dadurch gekennzeichnet, dass** der gesamte Drehmomentschlüssel **(1)** mit Kopfbereich **(10),** Halsbereich **(11),** Klinke **(4),** Schaftbereich **(12),** Indikatorbereich **(13),** Griffbereich **(14),** Basisast **(6)** und Biegeast **(7):**
a) aus einem Stück hergestellt ist; oder
b) die Klinke **(4)** als separates Bauteil am Drehmomentschlüssel **(1)** fixiert ist.

10. Drehmomentschlüssel **(1)** nach zumindest einem der Ansprüche 1 bis 9, **dadurch gekennzeichnet, dass** der Drehmomentschlüssel **(1):**
a) zumindest im wesentlichen aus einem Material der Gruppe, welche Edelstahl, Titan, Keramik und Kunststoff umfasst, hergestellt ist; und
b) zumindest im wesentlichen mittels eines Verfahrens der Gruppe, welche Laser- und Wasserstrahlschneiden, Drahterodieren, Fräsen, Stanzen, Spritzgiessen, Metalldruckguss umfasst, hergestellt ist.

## Claims

1. A torque wrench **(1)** as a ratchet instrument for the medical field, having:
a) a head region **(10)** located at the front, an adjoining neck region **(11),** which is followed by a shank region **(12),** and a handle region **(14)** arranged at the rear, these regions extending in the plane **(H);**
b) an accommodating opening **(3)** which is provided in the head region **(10)** and:
ba) is enclosed by a surround **(2);**
bb) has a centre point through which an axis **(V)** extends vertically, at least in principle; and
bc) serves for the insertion of a conventional screwing-in instrument **(9)** along the extent of the axis **(V),** which is perpendicular to the plane **(H);**
c) a catch segment **(40)** which is arranged on the periphery of the accommodating opening **(3),** can be moved to a limited extent and the front portion **(41-44)** of which is oriented toward the accommodating opening **(3),** the front portion **(41-44)** being intended:
ca) upon actuation of the torque wrench **(1)** in the forward direction, that is to say in screwing-in mode, for coming into carry-along engagement with an outer contour **(93,94;93',94')** provided on the head **(90)** of the screwing-in instrument **(9);**
cb) upon actuation of the torque wrench **(1)** in the return direction, that is to say in the ratchet mode, for releasing the carry-along engagement with the outer contour **(93,94;93',94')** provided on the head **(90)** of the screwing-in instrument **(9);** and
d) a flexurally rigid basic branch **(6)** which runs along the torque wrench **(1)** from the neck region **(11);**
e) a deflectable, preferably linearly elastic flexible branch **(7)** via which, upon actuation of the torque wrench **(1)** in the forward direction, the torque which is to be generated is introduced by means of a forward force **(F)** exerted by the user;
f) a catch spring **(47)** extends into the neck region **(11)** from the catch segment **(40),** the catch segment **(40)** and the catch spring **(47)** forming a single-piece catch **(4)** or one which is made up of a number of parts;
g) the catch segment **(40)** and catch spring **(47)** are arranged in the channel-like free space **(5),** which allows both to be deflected in the plane **(H)** counter to the force of the catch spring **(47);** and
h) the catch **(4)** is formed integrally from the neck region **(11)** or is fastened thereon as a separate component, **characterized in that**
i) the catch segment **(40)** has, on its front portion **(41-44),** a front nose **(44),** to one side of which is located a second flank **(42)** and to the other side of which is located a third flank **(43);**
j) the free space **(5)** opens out into the accommodating opening **(3)** by way of a first and a second stop **(50,51);**
k) the first stop **(50)** is arranged opposite the second flank **(42)** and, upon actuation of the torque wrench **(1)** in the forward direction, the second flank **(42)** of the catch **(4)** butts against the first stop **(50),** the front nose **(44)** being spaced apart at least in principle by the minimum distance **(a₀)** from the axis **(V);** and
l) the second stop **(51),** at least in part, is arranged opposite the third flank **(43)** and, upon actuation of the torque wrench **(1)** in the return direction, the third flank **(43)** of the deflected catch **(4)** has moved toward the second stop **(51)** or butts against the same, in which the front nose **(44)** is spaced apart by the maximum distance **(a₁)** from the axis **(V),** the second flank **(42)** is remote from the first stop **(50)** and the catch spring **(47)** has been elastically deformed counter to its restoring force.

2. The torque wrench **(1)** as claimed in claim 1, **characterized in that**
a) the catch spring **(47):**
aa) on the one hand, merges into the catch segment **(40)** at a spring outlet **(46)** and, on the other hand, merges into the neck region **(11)** at a spring mouth **(48);** and
ab) is in the form of a rectilinear or at least partially curved leaf spring;
b) the front nose **(44)** of the catch segment **(40):**
ba) is followed, in one direction, by a first flank **(41),** this being adjoined by the second flank **(42);**
bb) is followed, in the other direction, by the third flank **(43),** which extends in a rounded manner in the direction of the spring outlet **(46);**
bc) the front nose **(44)** being designed as a point or convexity which is raised in the direction of the accommodating opening **(3);**
c) the first stop **(50)** is formed, at least in principle, as a straight edge and the second stop **(51)** is of trough-like form; and
d) the catch spring **(47),** which projects through the free space **(5),** divides off a first and a second spring clearance **(52,52'),** the proportions of which change as the catch spring **(47)** moves, and which terminate in a base **(53)** which is followed by the spring mouth **(48).**

3. The torque wrench **(1)** as claimed in at least one of claims 1 and 2, **characterized in that**
a) the basic branch **(6)** extends, in the first instance as a longitudinal leg **(60),** from a transition **(61)** located in the neck region **(11),** via the shank region **(12),** into an indicator region **(13)** and terminates freely in the handle region **(14)** by way of a basic handle **(66);**
b) the flexible branch **(7)** runs, in the first instance as an elongate longitudinal leg **(70)** and in principle parallel to the longitudinal leg **(60),** into the indicator region **(13)** from a transition **(71)** located in the neck region **(11),** and terminates freely in the handle region **(14)** by way of a handle part **(76),** which projects beyond the basic handle **(66);**
c) the flexible branch **(7)** may have on its top side, directed toward the basic branch **(6),** a depression **(760),** in which the stop leg **(65)** and the basic handle **(66)** are partially embedded; and
d) a cutout **(8)** is located between the longitudinal leg **(60)** of the basic branch **(6)** and the longitudinal leg **(70)** of the flexible branch **(7)** and extends into the indicator region **(13)** from a groove base **(81)** adjacent to the transitions **(61,71).**

4. The torque wrench **(1)** as claimed in at least one of claims 1 to 3, **characterized in that**
a) the indicator region **(13)** is formed by:
aa) at least one branching portion **(62,63,64)** of the basic branch **(6),** the portion being located between the longitudinal leg **(60)** and the basic handle **(66);** and
ab) at least one branching portion **(72,73)** of the flexible branch **(7),** this branching portion being located between the longitudinal leg **(70)** and the handle part **(76);** and
b) as the torque wrench **(1)** is actuated in the forward direction and the flexible branch **(7)** is deflected, the relative positioning between the at least one branching portion **(62,63,64)** of the basic branch **(6)** and the at least one branching portion **(72,73)** of the flexible branch **(7),** starting from the rest position as a zero position, undergoes an incremental change which is a measure of the torque generated.

5. The torque wrench **(1)** as claimed in claim 4, **characterized in that** a first and a second measuring marking **(69,79)** are respectively provided on the at least one branching portion **(62,63,64)** of the basic branch **(6)** and the at least one branching portion **(72,73)** of the flexible branch **(7),** it being possible to read from these measuring markings the deflection of the flexible branch **(7),** due to the forward force **(F)** acting thereon, as the torque generated.

6. The torque wrench **(1)** as claimed in claim 4, **characterized in that** the indicator region **(13)** comprises:
a) a bracket **(670)** which extends from the longitudinal leg **(60)** to the basic handle **(66),** is formed by the at least one branching portion **(62,63,64)** and encloses a clearance **(67)** for which a through-passage **(68)** is provided; and
b) the at least one branching portion **(72,73)** of the flexible branch **(7),** this branching portion projecting through the through-passage **(68)** into the clearance **(67).**

7. The torque wrench **(1)** as claimed in claim 4, **characterized in that**
a) the bracket **(670)** is made up in meandering form from the first basic branching portion **(62),** which bends off from the longitudinal leg **(60)** in an L-shaped manner, and the consecutively adjoining second basic branching portion **(63)** and third basic branching portion **(64),** the latter merging into the basic handle **(66)** in an L-shaped manner;
b) the first branching portion **(72)** is adjoined in an L-shaped manner by a second branching portion **(73);**
c) a stop leg **(65)** extends inward, in the direction of the longitudinal leg **(60),** from the basic handle **(66)** and bounds the through-passage **(68)** on one side; wherein
d) the clearance **(67),** the stop leg **(65)** and the through-passage **(68),** as more or less stationary elements, and the first branching portion **(72)** with the second branching portion **(73),** as elements which can be deflected by way of the flexible branch **(7),** are dimensioned such that the maximum deflection of the flexible branch **(7)** is defined by the second branching portion **(73)** being positioned on the stop leg **(65).**

8. The torque wrench **(1)** as claimed in claim 4, **characterized in that**
aa) the first measuring marking **(69)** is provided in the form of a measurement scale, e.g. with the range 0 Ncm to 40 Ncm, at least on one of the basic branching portions **(62,64),** preferably on the basic branching portion **(64)** adjacent to the basic handle **(66);** and
ab) the second measuring marking **(79)** is provided in the form of an indicator element, e.g. as a reference line or arrow, on the first or second branching portion **(72,73)** of the flexible branch **(7),** preferably on the freely terminating, second branching portion **(73);** or
ba) the first measuring marking **(69)** is provided in the form of an indicator element, e.g. as a reference line or arrow, on the stop leg **(65)** of the basic branch **(6),** in the direction of the first branching portion **(72)** of the flexible branch **(7);** and
bb) the second measuring marking **(79)** is provided in the form of a measurement scale, e.g. with the range 0 Ncm to 40 Ncm, on the first branching portion **(72).**

9. The torque wrench **(1)** as claimed in at least one of claims 1 to 8, **characterized in that** the entire torque wrench **(1)** with head region **(10),** neck region **(11),** catch **(4),** shank region **(12),** indicator region **(13),** handle region **(14),** basic branch **(6)** and flexible branch **(7):**
a) is produced in one piece; or
b) the catch **(4)** is fixed as a separate component on the torque wrench **(1).**

10. The torque wrench **(1)** as claimed in at least one of claims 1 to 9, **characterized in that** the torque wrench **(1):**
a) is produced, at least essentially, from a material from the group comprising stainless steel, titanium, ceramic material and plastic; and
b) is produced, at least essentially, by means of a method from the group comprising laser cutting and water-jet cutting, wire-cut EDM, milling, punching, injection molding and metal diecasting.

## Revendications

1. Clé dynamométrique **(1)** en tant qu'instrument à cliquet pour la technique médicale, comprenant:
a) une région de tête **(10)** située la plus en avant, suivie d'une région de col **(11),** qui est suivie d'une région de tige **(12),** et une région de préhension **(14)** disposée la plus en arrière, lesquelles régions s'étendent dans le plan **(H);**
b) une ouverture de réception **(3)** prévue dans la région de tête **(10),** qui:
ba) est entourée par une enveloppe **(2);**
bb) possède un centre à travers lequel s'étend un axe **(V)** au moins en principe perpendiculairement; et
bc) sert à l'insertion d'un instrument de vissage usuel **(9)** dans l'étendue de l'axe **(V),** qui est perpendiculaire au plan **(H);**
c) un segment de cliquet **(40)** déplaçable de manière limitée et disposé sur la périphérie de l'ouverture de réception **(3),** dont la partie frontale **(41-44)** est tournée vers l'ouverture de réception **(3),** la partie frontale **(41-44)** étant prévue pour:
ca) lors de l'actionnement de la clé dynamométrique **(1)** dans la direction d'avance, c'est-à-dire en mode de vissage, venir en engagement d'entraînement avec un contour extérieur **(93,94;93',94')** prévu sur la tête **(90)** de l'instrument de vissage **(9);**
cb) lors de l'actionnement de la clé dynamométrique (1) dans la direction de recul, c'est-à-dire en mode de cliquet, desserrer l'engagement d'entraînement avec le contour extérieur **(93,94;93',94')** prévu sur la tête **(90)** de l'instrument de vissage **(9);** et
d) une branche de base **(6)** rigide en flexion, s'étendant depuis la région de col **(11)** le long de la clé dynamométrique **(1);**
e) une branche flexible **(7)** pouvant être déviée, de préférence élastique linéairement, par le biais de laquelle on introduit, lors de l'actionnement de la clé dynamométrique **(1)** dans la direction d'avance, le couple à générer au moyen d'une force d'avance **(F)** exercée par l'utilisateur;
f) un ressort de cliquet **(47)** s'étendant depuis le segment de cliquet **(40)** dans la région de col **(11),** le segment de cliquet **(40)** et le ressort de cliquet **(47)** formant un cliquet **(4)** assemblé d'une seule pièce ou constitué de plusieurs parties;
g) le segment de cliquet **(40)** et le ressort de cliquet **(47)** sont disposés dans un espace de jeu **(5)** en forme de canal qui permet la déviation des deux éléments dans le plan **(H)** à l'encontre de la force du ressort de cliquet **(47);** et
h) le cliquet **(4)** est formé d'une seule pièce dans la région de col **(11)** ou est fixé sur celle-ci sous forme de composant séparé, **caractérisée en ce que**
i) le segment de cliquet **(40)** présente sur sa partie frontale **(41-44)** un nez frontal **(44),** d'un côté duquel est disposé un deuxième flanc **(42)** et de l'autre côté duquel est disposé un troisième flanc **(43);**
j) l'espace de jeu **(5)** ouvre, avec une première et une deuxième butée **(50,51),** vers l'ouverture de réception **(3);**
k) la première butée **(50)** est disposée à l'opposé du deuxième flanc **(42)** et, lors de l'actionnement de la clé dynamométrique **(1)** dans la direction d'avance, le deuxième flanc **(42)** du cliquet **(4)** bute contre la première butée **(50),** le nez frontal **(44)** étant au moins en principe à une distance minimale **(a₀)** de l'axe **(V);** et
l) la deuxième butée **(51)** est disposée à l'opposé du troisième flanc **(43),** au moins en partie, et, lors de l'actionnement de la clé dynamométrique **(1)** dans la direction de recul, le troisième flanc **(43)** du cliquet dévié **(4)** se rapproche de la deuxième butée **(51)** ou vient buter contre celle-ci, le nez frontal **(44)** étant à la distance maximale **(a₁)** de l'axe **(V),** le deuxième flanc **(42)** étant éloigné de la première butée **(50)** et le ressort de cliquet **(47)** étant déformé élastiquement à l'encontre de sa force de rappel.

2. Clé dynamométrique **(1)** selon la revendication 1, **caractérisée en ce que**
a) le ressort de cliquet **(47):**
aa) est d'une part transféré dans une sortie de ressort **(46)** au segment de cliquet **(40)** et d'autre part transféré dans une embouchure de ressort **(48)** dans la région de col **(11);** et
ab) présente la forme d'un ressort à lame droit ou au moins en partie cintré;
b) au niveau du nez frontal **(44)** du segment de cliquet **(40):**
ba) dans une direction est disposé un premier flanc **(41)** auquel se raccorde de manière juxtaposée le deuxième flanc **(42);**
bb) dans l'autre direction est disposé le troisième flanc **(43),** qui s'étend sous forme arrondie dans la direction de la sortie de ressort **(46);**
bc) le nez frontal **(44)** est réalisé sous forme de pointe ou de bombement saillant dans la direction de l'ouverture de réception **(3);**
c) la première butée **(50)** est réalisée au moins en principe sous forme d'arête droite et la deuxième butée **(51)** est réalisée sous forme de creux; et
d) le ressort de cliquet **(47)** traversant l'espace de jeu **(5)** divise un premier et un deuxième espace libre de ressort **(52,52'),** dont les proportions varient avec le mouvement du ressort de cliquet **(47)** et se terminent dans un fond **(53)** au niveau duquel est placée l'embouchure de ressort **(48).**

3. Clé dynamométrique **(1)** selon au moins l'une quelconque des revendications 1 et 2, **caractérisée en ce que**
a) la branche de base **(6)** s'étend depuis une transition **(61)** située dans la région de col **(11)** d'abord sous forme de branche longitudinale **(60)** sur la région de tige **(12)** jusque dans une région d'indicateur **(13)** et se termine librement dans la région de préhension **(14)** avec une prise de base **(66);**
b) la branche flexible **(7)** s'étend depuis une transition **(71)** située dans la région de col **(11)** en principe parallèlement à la branche longitudinale **(60)** tout d'abord sous forme de branche longitudinale allongée **(70)** jusque dans la région d'indicateur **(13)** et se termine librement dans la région de préhension **(14)** avec une partie de préhension **(76)** qui dépasse la prise de base **(66);**
c) la branche flexible **(7)** peut présenter, au niveau de son côté supérieur tourné vers la branche de base **(6),** un renfoncement **(760)** dans lequel la branche de butée **(65)** et la prise de base **(66)** sont en partie encastrées; et
d) entre la branche longitudinale **(60)** de la branche de base **(6)** et la branche longitudinale **(70)** de la branche flexible **(7)** est pratiqué un évidement **(8)** qui s'étend depuis un creux de cannelure **(81)** adjacent aux transitions **(61,71)** jusque dans la région d'indicateur **(13).**

4. Clé dynamométrique **(1)** selon au moins l'une quelconque des revendications 1 à 3, **caractérisée en ce que**
a) la région d'indicateur **(13)** est formée par:
aa) au moins une dérivation **(62,63,64)** de la branche de base **(6),** qui se situe entre la branche longitudinale **(60)** et la prise de base **(66);** et
ab) au moins une dérivation **(72,73)** de la branche flexible **(7),** qui se situe entre sa branche longitudinale **(70)** et la partie de préhension **(76);** et
b) avec l'actionnement de la clé dynamométrique **(1)** dans la direction d'avance et la déviation de la branche flexible **(7),** la position relative entre l'au moins une dérivation **(62,63,64)** de la branche de base **(6)** et l'au moins une dérivation **(72,73)** de la branche flexible **(7),** partant de la position de repos avec une position nulle, subit une variation incrémentale qui constitue une mesure pour le couple généré.

5. Clé dynamométrique **(1)** selon la revendication 4, **caractérisée en ce que** sur l'au moins une dérivation **(62,63,64)** de la branche de base **(6)** et sur l'au moins une dérivation **(72,73)** de la branche flexible **(7)** sont réalisés un premier ou un deuxième marquage de mesure **(69,79),** au niveau desquels peut être lue la déviation de la branche flexible **(7)** résultant de l'application de la force d'avance **(F)** en tant que couple généré.

6. Clé dynamométrique (1) selon la revendication 4, **caractérisée en ce que** la région d'indicateur (13) se compose:
a) d'un étrier **(670)** s'étendant depuis la branche longitudinale **(60)** jusqu'à la prise de base **(66),** lequel est formé par l'au moins une dérivation **(62,63,64)** et entoure un espace libre **(67),** vers lequel est prévu un passage **(68);** et
b) de l'au moins une dérivation **(72,73)** de la branche flexible **(7)** qui pénètre à travers le passage **(68)** à l'intérieur de l'espace libre **(67).**

7. Clé dynamométrique **(1)** selon la revendication 4, **caractérisée en ce que**
a) l'étrier **(670)** se compose, en forme de méandres, de la première dérivation de base **(62)** coudée en forme de L depuis la branche longitudinale **(60)** et de la deuxième dérivation de base **(63)** suivante se raccordant à celle-ci et de la troisième dérivation de base **(64)** qui se prolonge en forme de L dans la prise de base **(66);**
b) une deuxième dérivation **(73)** se raccorde en forme de L à la première dérivation **(72);**
c) une branche de butée **(65)** s'étend depuis la prise de base **(66)** vers l'intérieur dans la direction de la branche longitudinale **(60)** et délimite d'un côté le passage **(68);**
d) l'espace libre **(67),** la branche de butée **(65)** et le passage **(68)** sont dimensionnés en tant qu'éléments quasiment fixes et la première dérivation **(72)** avec la deuxième dérivation **(73)** sont dimensionnées en tant qu'éléments pouvant être déviés avec la branche flexible **(7),** de telle sorte que la déviation maximale de la branche flexible **(7)** soit définie par l'application de la deuxième dérivation **(73)** sur la branche de butée **(65).**

8. Clé dynamométrique **(1)** selon la revendication 4, **caractérisée en ce que**
aa) le premier marquage de mesure **(69)** est prévu sous la forme d'une échelle de mesure, par exemple avec une plage de 0 Ncm à 40 Ncm, au moins sur l'une des dérivations de base **(62,64),** de préférence sur la dérivation de base **(64)** adjacente à la prise de base **(66);** et
ab) le deuxième marquage de mesure **(79)** est appliqué sous la forme d'un élément indicateur, par exemple sous la forme d'un trait de référence ou d'une flèche, sur la première ou la deuxième dérivation **(72,73)** de la branche flexible **(7),** de préférence sur la deuxième dérivation **(73)** à extrémité libre; ou
ba) le premier marquage de mesure **(69)** est appliqué sous la forme d'un élément indicateur, par exemple sous la forme d'un trait de référence ou d'une flèche, sur la branche de butée **(65)** de la branche de base **(6),** dans la direction de la première dérivation **(72)** de la branche flexible **(7);** et
bb) le deuxième marquage de mesure **(79)** est prévu sous la forme d'une échelle de mesure, par exemple avec une plage de 0 Ncm à 40 Ncm, sur la première dérivation **(72).**

9. Clé dynamométrique **(1)** selon au moins l'une quelconque des revendications 1 à 8, **caractérisée en ce que** l'ensemble de la clé dynamométrique **(1)** avec la région de tête **(10),** la région de col **(11),** le cliquet **(4),** la région de tige **(12),** la région d'indicateur **(13),** la région de préhension **(14),** la branche de base **(6)** et la branche flexible **(7):**
a) est fabriqué d'une seule pièce; ou
b) le cliquet **(4)** est fixé sous forme de composant séparé sur la clé dynamométrique **(1).**

10. Clé dynamométrique **(1)** selon au moins l'une quelconque des revendications 1 à 9, **caractérisée en ce que** la clé dynamométrique **(1):**
a) est fabriquée au moins essentiellement en un matériau du groupe comprenant l'acier spécial, le titane, la céramique et le plastique; et
b) est fabriquée au moins essentiellement au moyen d'un procédé du groupe comprenant le découpage au laser et le découpage au jet d'eau, l'électroérosion par fil, le fraisage, l'estampage, le moulage par injection, et la coulée de métal sous pression.
